# EUROPÄISCHE PATENTSCHRIFT

(11) **EP 2 921 276 B1**
(45) Veröffentlichungstag und Bekanntmachung des Hinweises auf die Patenterteilung: **19.02.2020**
(21) Anmeldenummer: 15166337.4
(22) Anmeldetag: 03.03.2010
(51) Int. Cl.: B29C 43/48, C08J 5/18, C08L 3/00, A61J 3/07, A61K 9/48, B29C 43/00, B29C 43/44, C08L 3/02, C08L 3/04, C08L 3/08

(54) **WEICHKAPSEL AUF STÄRKEBASIS SOWIE VERFAHREN UND VORRICHTUNG ZUR HERSTELLUNG DERSELBEN**
SOFT CAPSULE CAPSULES BASED ON STARCH, AND METHOD AND DEVICE FOR MAKING SAME
CAPSULE SOUPLE À BASE D'AMIDON ET PROCÉDÉ ET DISPOSITIF DE FABRICATION DE LA PLAQUE DE CONSTRUCTION

(30) Priorität: 03.03.2009 CH 3242009
(43) Veröffentlichungstag der Anmeldung: 23.09.2015
(62) Teilanmeldung aus: 10707269.6
(73) Patentinhaber: Innogel AG, 6331 Hünenberg (CH)
(72) Erfinder: Müller, Rolf, 8055 Zürich (CH); Innerebner, Federico, 8049 Zürich (CH)
(74) Vertreter: Becker & Kurig Partnerschaft Patentanwälte PartmbB

(56) Entgegenhaltungen:
- EP-A1- 0 546 538
- EP-A1- 0 546 539
- WO-A1-01/03677
- WO-A1-2007/128150
- WO-A1-2008/105662
- WO-A2-2004/085483
- DE-A1- 19 943 604
- US-A- 3 098 492
- US-A- 3 661 154
- LAI L S ET AL: "PHYSICOCHEMICAL CHANGES AND RHEOLOGICAL PROPERTIES OF STARCH DURING EXTRUSION (A REVIEW)", BIOTECHNOLOGY PROGRESS, AMERICAN INSTITUTE OF CHEMICAL ENGINEERS, US LNKD- DOI:10.1021/BP00009A009, Bd. 7, Nr. 3, 1. Januar 1991 (1991-01-01), Seiten 251-266, XP009086522, ISSN: 8756-7938
- H.A. PUSHPADAS ET AL.: "Macromolecular Changes in extruded Starch-Films Plasticized with Glycerol, Water and Stearic Acid", STARCH/STRÄRKE, Bd. 61, 13. Mai 2009 (2009-05-13), Seiten 256-266, XP002591655, DOI: 10.1002/star.200800046
- D. PERESSINI ET AL.: "Starch-Methylcellulose based edible films; rheological properties of film-forming dispersions", JOURNAL OF FOOD ENGINEERING, Bd. 59, 2003, Seiten 25-32, XP002591656,

## Beschreibung

Die vorliegende Erfindung bezieht sich auf ein Verfahren, insbesondere ein Gießverfahren, zur Herstellung von Stärkeweichkapseln, auf die daraus erhaltenen Weichkapseln, sowie auf eine Vorrichtung zur Herstellung von erfindungsgemäßen Weichkapseln.

### Stand der Technik

Weichkapseln werden beispielsweise zur Aufnahme von pharmazeutischen Wirkstoffen, diätetischen Produkten und Nahrungsergänzungsmitteln verwendet. In der Regel besteht die Hülle von Weichkapseln hauptsächlich aus Gelatine, weshalb die Kapseln auch häufig als Weichgelatinekapseln bezeichnet werden. Zwar wird fast ausschliesslich Gelatine eingesetzt, doch weist dieser Stoff zahlreiche Nachteile auf. Gelatine ist ein Material tierischen Ursprungs und wurde damit anlässlich der BSE Krise das erste mal Gegenstand von Bedenken und öffentlicher Kritik. Seither wird intensiv nach Alternativen auf pflanzlicher Basis gesucht. Nach der BSE Krise entstanden im Umfeld der wiederkehrenden Fleischskandale immer wieder von neuem Bedenken und verstärkte sich generell der Trend hin zu vegetarischen Lösungen. Gelatine Kapseln sind darum bei Vegetarieren unerwünscht und bei Veganern unakzeptabel. Da Gelatine grossteils aus Schlachtabfällen von Schweinen gewonnen wird, sind Gelatine Kapseln ausserdem für Konsumenten die kosher oder halal Produkte benötigen, nicht akzeptabel.

Der erwünschte Gelatineersatz im Bereich der Weichkapseln sollte vorzugsweise pflanzlichen Ursprungs sein, Kapseln mit der bekannten Qualität von Gelatine ermöglichen, der alternative Rohstoff sollte nicht teuerer sein, das Verfahren nicht aufwendiger werden und die neue Technologie dürfte keine grösseren Investitionen benötigen.

In der Patentschrift US 6,770,293 werden Hüllen von Weichkapseln auf Basis von Copolymeren aus Polyvinylestern und Polyestern vorgeschlagen. Dies sind jedoch synthetische Polymere. Im Bereich der Weichkapsel gibt es hingegen den Wunsch, die bisher vorherrschende Gelatine durch alternative Stoffe auf pflanzlicher Basis zu ersetzen.

In der Patentschrift US 5,342,626 wird die Herstellung von gelatinefreien Weichkapseln beschrieben, welche auf einer Mischung mit den wesentlichen Bestandteilen Gellan, Carrageenan und Mannan basiert. Die beschriebene Mischung verhält sich ähnlich wie Gelatine, so dass sie bei hohen Temperaturen flüssig ist und bei Erkaltung einen Film bildet. Auch in der Patentschrift US 6,949,256 wird die Herstellung von gelatinefreien Weichkapseln auf der Basis von Carrageenan beschrieben. Auf diese Weise hergestellte Weichkapseln basieren zwar auf pflanzlichen Ausgangsmaterialien, haben allerdings den Nachteil, dass Carrageenan teuer ist und unter dem Verdacht steht, krebserregend zu sein. Ausserdem sind entsprechende Weichkapseln aufgrund der hohen Viskosität der Schmelze und der langsamen Geliergeschwindigkeit von Carrageenan nur mit einem schwierigen, empfindlichen Verfahren herzustellen und weisen gegenüber Gelatineweichkapseln deutlich schlechtere Eigenschaften auf.

Ein weiteres Material zur Herstellung von Weichkapseln, welches aus pflanzlichen Quellen stammt, ist Stärke. Bei Stärke handelt es sich um einen deutlich billigeren Rohstoff als Gelatine. Gießverfahren zur Herstellung von Stärkeweichkapseln aus wässerigen Stärkelösungen werden allerdings durch den notwendigerweise tiefen Stärke- und hohen Wasseranteil solcher Lösungen limitiert. Mischungen von destrukturierter oder gelöster Stärke werden bei typischerweise 5% Stärke bereits so viskos, dass zumindest einfache Gießverfahren nicht mehr möglich sind. Ursache ist das sehr hohe Molekulargewicht von Stärke, das bis 100.000.000 g/mol betragen kann.

Indem die eingesetzte Stärke gegebenenfalls hydrolysiert, somit das Molekulargewicht reduziert wird und die Stärkekörner durch Kochen und/oder Scherung aufgelöst werden, können auch Mischungen mit Stärke von höheren Konzentrationen gegossen werden. Allerdings kann dann beim Gießen keine Gelierung erhalten werden, die Lösung wird vielmehr durch Abkühlen und langsames Trocknen allmählich verfestigt, sodass die Produktionsrate sehr gering bleibt. Ausserdem werden auch schlechtere mechanische Eigenschaften der Weichkapseln erhalten, da die langen Stärkemakromoleküle die mechanischen Eigenschaften positiv beeinflussen. Beispielsweise werden in der US 6,375,981 gegossene Stärkeweichkapseln beschrieben. Die verwendete hydrolysierte Stärke wird unter Bedingungen gekocht, welche zu einer vollständigen Zerstörung der Stärkekörner führen.

Üblicherweise werden Stärkeweichkapseln allerdings mittels Extrusion erhalten, wozu teure Extruder benötigt werden. Dabei wird aus der typischerweise granulär vorliegenden Stärke bei Temperaturen von mehr als 100°C bei der Plastifizierung unter der Einwirkung von mechanischer Energie in Form von Scherung eine homogene Stärkeschmelze von hoher Viskosität hergestellt, welche unter hohen Drücken durch eine schlitzförmige Düse gepresst wird, um zunächst einen Stärkefilm zu erzeugen. Infolge des hohen mechanischen Energieeintrags wird das Molekulargewicht der Stärke deutlich reduziert, was für die mechanischen Eigenschaften des Films nachteilig ist und ausserdem werden die Makromoleküle bei den Fliessvorgängen an der Düse in Längsrichtung des Films orientiert, sodass der Film anisotrop ist, was für die Weiterverarbeitung nachteilig ist. Bei der Extrusion von Stärkefilmen findet nach der Formgebung keine Gelierung statt, vielmehr sinkt nach der Formgebung die Temperatur im Film, wodurch die Festigkeit des Films etwas zunimmt. Dieser Film wird dann zu Weichkapselhüllen weiterverarbeitet.

Weichkapseln, die aus homogenen Stärkefilmen hergestellt werden, die mittels Extrusion erhalten werden, sind beispielsweise aus der EP 1 103 254 B1 bekannt. Sie sind wegen der benötigten Extruder aufwändig in der Herstellung und die plastifizierten Stärke Filme sind schwierig zu verschweissen, weshalb hohe Schweisstemperaturen benötigt werden. Die erhaltenen Kapseln weisen schlechte Eigenschaften auf, insbesondere sind sie bei tiefer Luftfeuchtigkeit brüchig.

Eine Destrukturierung von Stärke wird durch Erhitzen von Stärke in einem wässrigen Medium erhalten, wobei mit zunehmender Temperatur die Destrukturierung zunimmt. Werden die Stärkekörner gleichzeitig durch Scherkräfte mechanisch beansprucht, so wird bei gleicher Temperatur eine höhere Destrukturierung erhalten. Ist die Kristallinität der Stärkekörner substanziell zerstört, so reichen schon geringe Scherkräfte, wie sie z. B. bei einfachen Misch- und Fliessvorgängen von Stärkemischungen auftreten, um den Destrukturierungsgrad weiter zu erhöhen und die gequollenen Stärkekörner substanziell zu zerstören, wobei ausserdem auch das Molekulargewicht der Stärke Makromoleküle deutlich reduziert werden kann. Der Grad der Destrukturierung lässt sich in folgende Stufen unterteilen:
Stufe 1: die Kristallinität der Stärke ist teilweise zerstört, im Polarisationsmikroskop sind
Stufe 1.1: höchstens 5% der Körner nicht mehr doppelbrechend
Stufe 1.2: 5 - 10% der Körner nicht mehr doppelbrechend
Stufe 1.3: 10 - 20% der Körner nicht mehr doppelbrechend
Stufe 1.4: 20 - 30% der Körner nicht mehr doppelbrechend
Stufe 1.5: 30 - 40% der Körner nicht mehr doppelbrechend
Stufe 2: die Kristallinität der Stärke ist substantiell zerstört, im Polarisationsmikroskop sind
Stufe 2.1: 40 - 50% der Körner nicht mehr doppelbrechend
Stufe 2.2: 50 - 60 % der Körner nicht mehr doppelbrechend
Stufe 2.3: 60 - 80% der Körner nicht mehr doppelbrechend
Stufe 2.4: 80 - 100% der Körner sind nicht mehr doppelbrechend
Stufe 3: es sind höchstens 5% der Körner doppelbrechend
Stufe 3.1: und 1 - 10% der Körner sind aufgeplatzt
Stufe 3.2: und 10 - 20% der Körner sind aufgeplatzt
Stufe 3.3: und 20 - 30% der Körner sind aufgeplatzt
Stufe 3.4: und 30 - 50% der Körner sind aufgeplatzt
Stufe 3.5: und 50 - 70% der Körner sind aufgeplatzt
Stufe 3.6: und 70 - 100% der Körner sind aufgeplatzt

Aufgeplatzte Stärkekörner sind dadurch charakterisiert, dass die Stärkekörner an der Oberfläche Risse aufweisen und/oder die zuvor relativ glatte Oberfläche deutlich deformiert worden ist (z.B. schrumpelige Oberfläche). Neben noch als ganze Körner vorliegenden Stärkepartikeln können auch in Bruchstücke zerfallene Stärkepartikel vorliegen. Die Stärkekörner wie auch die Bruchstücke sind jedoch noch als Entitäten erkennbar.
Stufe 4: Es wird keinerlei Doppelbrechung beobachtet, die Stärkekörner sind substanziell zerstört
Stufe 4.1: es liegen noch Fragmente von Stärkekörnern vor, die Stärke liegt grossteils gelöst vor
Stufe 4.2: die Stärke liegt vollständig gelöst vor

Der Begriff "destrukturierte Stärke" wird in der Fachwelt nicht einheitlich verwendet. Als destrukturierte Stärke wird hier eine Stärke bezeichnet, welche höchstens bis zur Stufe 4.1 destrukturiert worden ist, d.h. die Stärke liegt mindestens teilweise noch in Form von Partikeln vor.

Allen Stärkeweichkapseln, die aus Lösungen degradierter Stärke oder extrudierten Stärkemassen hergestellt wurden, ist gemein, dass das Molekulargewicht der Stärke deutlich gesenkt und die Stärkepartikel im wesentlichen vollständig zerstört wurden. Weichkapseln gemäß den erwähnten Veröffentlichungen US 6,375,981 und EP 1 103 254 B1 enthalten folglich im wesentlichen nur Stärke der Destrukturierungsstufe 4.2.

Aufgabe der vorliegenden Erfindung ist es, eine einfach und kostengünstig herzustellende Weichkapsel auf Basis von unbedenklichen und günstigen pflanzlichen Rohstoffen mit guten mechanischen Eigenschaften bereitzustellen.

Bevorzugt sollte die erfindungsgemäße Weichkapsel mit dem Standard Verkapselungsverfahren "Rotary Die" herstellbar sein.
Mit dem Begriff Weichkapsel wird sowohl die Weichkapsel als Ganzes, d. h. die Weichkapselhülle plus Inhalt, als auch die Weichkapselhülle alleine bezeichnet und ist im Text jeweils sinngemäss die Weichkapsel als Ganzes bzw. die Weichkapselhülle zu verstehen.

### Beschreibung der Erfindung

Diese Aufgabe wird erfindungsgemäß durch ein Verfahren zur Herstellung einer Weichkapsel auf Stärkebasis, insbesondere durch ein Gießverfahren gelöst, wobei eine Mischung enthaltend Stärke, wobei mehr als 50 Gew.-% der Stärke in der flüssigen Phase als Partikeln granulärer Stärke vorliegen, zu einem Film umgeformt wird und während und/oder nach dieser Umformung die Mischung durch eine Temperaturerhöhung, insbesondere von mehr als 5°C, verfestigt wird und aus diesem Film Weichkapseln hergestellt werden.

Bevorzugt wird diese Aufgabe durch ein Verfahren zur Herstellung einer Weichkapsel auf Stärkebasis, insbesondere ein Gießverfahren, gelöst, umfassend die folgenden Schritte:
- Herstellen einer Mischung, umfassend:
   a) > 40 Gew.-% der trockenen Mischung, nach Abzug des Weichmachers, Stärke, wobei mehr als 50 Gew.-% der Stärke in der flüssigen Phase als Partikeln granulärer Stärke vorliegen,
   b) 15 - 70 Gew.-% der trockenen Mischung Weichmacher,
   c) 15 - 90 Gew.-% der gesamten Mischung Wasser,
   d) gegebenenfalls maximal 50 Gew.-% der trockenen Mischung, nach Abzug des Weichmachers, ein Verdickungsmittel, und
   e) gegebenenfalls übliche Zusatz- und Hilfsstoffe,
- Umformen der Mischung zu einem Film in einem Formgebungsprozess,
- Verfestigen der Mischung durch Erhöhen der Temperatur der Mischung während und/oder nach dem Formgebungsprozess um mehr als 5°C, und
- Umformen des Films zu einer Weichkapsel, die Partikeln destrukturierter Stärke aufweist.

Erfindungsgemäß wird dabei das Molekulargewicht der Stärke nicht wesentlich beeinträchtigt. Dadurch sind besonders gute mechanische Eigenschaften des frischen Films, der frischen Weichkapsel und der getrockneten Weichkapsel möglich. Weiter aber trägt auch die heterogene Struktur des Materials wesentlich hierzu bei, indem die bei der Verfestigungstemperatur entstandenen, destrukturierten Partikel der granulären Stärke bereits eine gewisse Festigkeit und Elastizität per se aufweisen, was sich vorteilhaft auf die Handhabung des frischen Films und der frischen Weichkapseln wie auch auf die getrockneten Weichkapseln auswirkt.

In den Unteransprüchen sind vorteilhafte Ausführungsformen der Erfindung enthalten.

Für gute mechanische Eigenschaften des Stärkefilms sollten das Molekulargewicht der Stärke und ihr Anteil in der Ausgangsmischung gross genug sein. Die zunächst widersprüchliche Kombination von Gießfähigkeit, also von tiefer Viskosität und hohem Molekulargewicht der Stärke wird erfindungsgemäß dadurch erreicht, dass die Stärke in der Gießmischung Partikeln aufweist. Dann wird die Viskosität der Mischung primär von der Viskosität von Wasser und Weichmacher bestimmt und ist entsprechend tief. So ist beispielsweise eine Stärkemischung mit rund 35 Gew.-% Wasser insgesamt und 35 Gew.-% Glycerin, bezogen auf den Stärkeanteil noch gut, sogar drucklos gießbar. Eine Mischung gleicher Zusammensetzung, wobei jedoch die Stärke vor der Formgebung in destrukturierter, gelöster oder plastifizierter Form vorliegt, würde eine um mindestens 1000mal höhere Viskosität von mehr als 10.000 Pas aufweisen. Um eine solche Mischung zu einem Film umzuformen, würden hohe Drücke benötigt, wie sie beispielsweise von Extrudern erzeugt werden können. Alternativ müsste der Wassergehalt einer solchen Mischung auf rund 95% erhöht werden, damit die Mischung drucklos gießbar wird. Allerdings läge dann nach dem Gießen auch bei einer nachfolgenden Temperaturerhöhung immer noch eine Flüssigkeit vor und kein Film mit brauchbaren mechanischen Eigenschaften, der sich zu einer Weichkapsel weiterverarbeiten ließe.

Wird die Stärkemischung erhitzt, so findet eine zunehmende Quellung und Destrukturierung der Stärkepartikel statt, wobei die Partikel Wasser und Weichmacher aufnehmen, anquellen und miteinander verkleben. Es wird ein Agglomerat oder Konglomerat von Partikeln erhalten, also eine heterogene Struktur bestehend aus einem Gemenge von Stärkepartikeln.

Die Verfestigung der zuvor tiefviskosen, gießfähigen Mischung zu einem viskoselastischen, festen Material, das durch eine typische Festkörpereigenschaft wie den E-Modul charakterisiert werden kann, erfolgt nahezu simultan mit der Temperaturerhöhung und entsteht dadurch, dass die flüssige Phase von Wasser und Weichmacher verschwindet, d. h. in die Stärkepartikel eindiffundiert, innerhalb der Stärkepartikel durch Verschlaufungen von Makromolekülen eine Netzwerk-, d.h. eine Gelstruktur entsteht und die Partikel miteinander verklebt werden. Die Verklebung der Partikel kann durch die Zugabe eines Verdickungsmittels gegebenenfalls noch modifiziert werden.

Unter Verfestigung der Mischung ist die primäre Verfestigung zu verstehen, wobei eine Destrukturierung von granulärer Stärke, also eine Phasenumwandlung der Stärke stattfindet und sich die Eigenschaften der Mischung um Grössenordnungen ändern. Diese Phasenumwandlung zeigt sich als Gelierung der Gießmasse zu einem festen Film. Hierzu werden ausser Stärke keine weiteren Gelierungsmittel benötigt. Nach der primären Verfestigung der Mischung findet eine sekundäre Verfestigung statt, begleitet von einer graduellen Änderung der Stoffeigenschaften, wenn die Temperatur des primär verfestigten Films und/oder deren Wassergehalt reduziert wird und/oder indem eine langsame Netzwerkbildung der Stärke einsetzt, die durch Retrogradation, d. h. Kristallisation der Stärkemakromoleküle hervorgerufen wird.

Erfindungsgemäß können Mischungen von Stärke mit hohen Stärkeanteilen mit einfachen Gießverfahren zu Weichkapseln verarbeitet werden. Im Vergleich zu einer Mischung, in welcher dieselbe Menge Stärke in gelöster Form vorliegt, werden um Grössenordnungen tiefere Viskositäten der Mischung erhalten. Die Verfestigung der Mischung zu einem isotropen Weichkapselmaterial von hoher Elastizität und hohem Dehnvermögen wird durch eine Temperaturerhöhung erreicht. Vorteilhaft ist, dass das Molekulargewicht der Stärke im erfindungsgemäßen Verfahren nicht wesentlich beeinträchtigt wird. Bevorzugt ist der Quotient M_{w}2/M_{w}1 > 0,3, noch bevorzugter > 0,4, noch bevorzugter > 0,5, noch bevorzugter > 0,6, noch bevorzugter > 0,7, noch bevorzugter > 0,8, wobei M_{w}1 das Gewichtsmittel der Molekulargewichtsverteilung der eingesetzten Stärke und M_{w}2 das Gewichtsmittel der Molekulargewichtsverteilung der Stärke in der hergestellten Weichkapsel ist. Hier ist anzumerken, dass das Molekulargewicht von Stärke sehr empfindlich auf mechanische Beanspruchung reagiert. So wird beispielsweise das Molekulargewicht von gelöster Stärke durch blosses Schütteln der Lösung bereits messbar reduziert.

Die erfindungsgemäß eingesetzten Stärkemischungen können in nahezu gleicher Art und Weise zu Weichkapseln verarbeitet werden, wie dies bei Gelatinegießverfahren der Fall ist. Dies ist eine besonders günstige Voraussetzung, um Gelatineweichkapseln zu ersetzten, da dieselben Anlagen genutzt werden können. Der verfahrenstechnische Unterschied zu Gelatineweichkapseln besteht im Wesentlichen darin, dass Gelatineschmelzen bei Abkühlung gelieren, während erfindungsgemäße Stärkemischung infolge einer Temperaturerhöhung gelieren. Die erfindungsgemäßen Weichkapseln haben also nicht nur die bekannte Qualität von Gelatineweichkapseln, auch wird das bisherige Verfahren nicht aufwendiger und die neue Technologie benötigt keine grösseren Investitionen. Vielmehr fällt sogar die aufwendige Aufbereitung der Gelatineschmelze weg, bzw. kann durch einen einfachen Mischvorgang ersetzt werden.

### Stärke

Grundsätzlich können hinsichtlich des Ursprungs und Aufbereitung beliebige Stärken oder Mischungen davon eingesetzt werden. Sie können zum Beispiel im nativen Zustand, wie auch im physikalisch und/oder chemisch/enzymatisch modifizierten Zustand eingesetzt werden.

Hinsichtlich des Ursprungs sind Wurzelstärken wie beispielweise Kartoffelstärken oder Tapiocastärken bevorzugt, da diese im Vergleich zu Stärken anderer Herkunft niedrige Gelantinisierungstemperaturen aufweisen und die Verfestigung bzw. Gelierung der Gießmasse zu Filmen zur Weichkapselherstellung daher bereits bei niedrigen Temperaturen möglich ist. Besonders bevorzugt ist Tapiocastärke. Tapiocastärke ist farblos, geschmacklos, weist eine sehr gute Transparenz auf und es sind von Tapiocastärken noch keine genetisch modifizierten Varianten bekannt.

In einer bevorzugten Ausführung wird die Stärke im nativen, d.h. nicht modifizierten Zustand eingesetzt. Hiermit können gute Eigenschaften bei tiefen Kosten erhalten werden.

In einer weiteren bevorzugten Ausführungsform werden substituierte Stärken wie Stärkeester und Stärkeether eingesetzt wie beispielsweise hydroxypropylierte oder acetylierte Stärken. Diese Modifikationen führen zu besonders hoher Transparenz und hohem Dehnvermögen des Films.

Alternativ werden oxidierte Stärken eingesetzt.

In einer weiteren bevorzugten Ausführungsform werden vernetzte Stärken eingesetzt, insbesondere vernetzte Stärkeester bzw. vernetzte Stärkeether, beispielsweise Stärkephosphate und Stärkeadipate. Durch die Erhöhung des Molekulargewichtes, welche mit der Vernetzung einhergeht, werden verbesserte mechanische Eigenschaften erhalten und werden die Stärkekörner als Einheiten auch mechanisch stabilisiert, was für das Verfahren besonders vorteilhaft ist, da damit der Beitrag der Stärkepartikel zu den mechanischen Eigenschaften des frischen Films, der frischen Weichkapsel und der getrockneten Weichkapsel gesteigert werden kann. Bei hochvernetzten Stärken bildet das destrukturierte Stärkekorn praktisch ein Molekül von gigantischem Molekulargewicht und weist eine besonders hohe Stabilität auf.

In einer weiteren bevorzugten Ausführungsform wird substituierte Tapiocastärke eingesetzt, insbesondere vernetzte substituierte Tapiocastärke wie beispielsweise hydroxypropyliertes Stärkephosphat.

Das bevorzugte Gewichtsmittel der Molekulargewichtsverteilung M_{w}1 der verwendeten Stärke liegt mindestens bei 500.000 g/mol, besonders bevorzugt mindestens 1.000.000 g/mol, noch bevorzugter mindestens 2.500.000 g/mol, noch bevorzugter mindestens 3.000.000 g/mol, noch bevorzugter mindestens 4.000.000 g/mol, noch bevorzugter mindestens 5.000.000 g/mol, noch bevorzugter mindestens 7.000.000 g/mol, ganz besonders bevorzugt mindestens 10.000.000 g/mol.

Der Amylosegehalt der Stärken in Gew.-% ist bevorzugt < 50, noch bevorzugter < 40 noch bevorzugter < 35, noch bevorzugter < 30, noch bevorzugter < 27, noch bevorzugter < 25, ganz besonders bevorzugt < 20. Hohe Amylosegehalte führen zu reduziertem Dehnvermögen des Films und die resultierenden Weichkapseln sind bezüglich Glanz und Transparenz von minderer Qualität. Zudem verschlechtern sich ihre Zerfallseigenschaften in wässerigem Medium.

Weiterhin sind Waxy-Stärken bevorzugt, insbesondere vernetzte und/oder substituierte Waxy-Stärken. Waxy-Stärken sind betreffend die Transparenz vorteilhaft.

Der Amylosegehalt der Stärken in Gew.-% ist bevorzugt >= 0, noch bevorzugter > 0,3, noch bevorzugter > 0,5, noch bevorzugter > 0,7, noch bevorzugter > 1, noch bevorzugter > 2, ganz besonders bevorzugt > 3. Zu tiefe Amylosegehalte können zu reduziertem Dehnvermögen des Films führen.

Weiterhin bevorzugt sind Stärken mit einer Gelatinisierungstemperatur < 90°C, besonders bevorzugt < 80°C, noch bevorzugter < 75°C, noch bevorzugter < 70°C, noch bevorzugter < 67°C, ganz besonders bevorzugt < 65°C. Die Gelatinisierungstemperatur wird mittels DSC (Differential Thermal Calorimetry) als Peak-Temperatur beim Aufheizen mit 10°C/min von einer Stärke/Wasser-Mischung enthaltend 65 Gew.-% Wasser bestimmt. Mit abnehmender Gelatinisierungstemperatur wird die Verfestigung des gegossenen Films bei tieferen Temperaturen möglich und somit sowohl einfacher als auch schneller.

Weiter bevorzugt sind Stärken mit einem Dextrose-Äquivalent (DE) von < 10, besonders bevorzugt < 1, am bevorzugtesten < 0,7, noch bevorzugter < 0,5, noch bevorzugter < 0,2, noch bevorzugter < 0,1, ganz besonders bevorzugt < 0,05. Das Dextrose-Äquivalent eines Polysaccharid-Gemischs bezeichnet den prozentualen Anteil reduzierender Zucker an der Trockensubstanz. Es entspricht der Menge Glucose (= Dextrose), die je 100 g Trockensubstanz das gleiche Reduktionsvermögen hätte. Der DE-Wert ist ein Maß dafür, wie weit der Polymerabbau erfolgt ist, daher erhalten Produkte mit niedrigem DE-Wert einen hohen Anteil an Polysacchariden und einen niedrigen Gehalt an niedermolekularen Zuckern (Oligosaccharide), während Erzeugnisse mit hohem DE-Wert hauptsächlich nur noch aus niedermolekularen Zuckern bestehen. Das Dextrose-Äquivalent wird nach der ISO Norm 5377 bestimmt. Mit tiefer werdendem DE Wert nimmt die Festigkeit der Weichkapsel nach Verfestigung zu.

In einer bevorzugten Ausführungsform der Erfindung liegt der Stärkeanteil der trockenen Mischung, nach Abzug des Weichmachers (d. h. nach mathematischer Subtraktion des Weichmachers von der Trockenmischung, bestehend aus Stärke, Weichmacher und allen optionalen Bestandteilen), in Gew.-% bei > 40, besonders bevorzugt > 50, noch bevorzugter > 60, noch bevorzugter > 70, noch bevorzugter > 80, noch bevorzugter > 90, besonders bevorzugt > 95.

### Granuläre Stärke

Die granuläre Stärke wird bevorzugt mit einer Destrukturierung bis zur 2.2., noch bevorzugter bis zur Stufe 2.1, noch bevorzugter bis zur Stufe 1.5, noch bevorzugter bis zur Stufe 1.4, noch bevorzugter bis zur Stufe 1.3, noch bevorzugter bis zur Stufe 1.2, noch bevorzugter bis zur Stufe 1.1, ganz besonders bevorzugt im nativen, nicht destrukturierten Zustand eingesetzt. Mit abnehmender Destrukturierung nimmt die Viskosität der Mischung ab, wodurch das Gießen vereinfacht wird.

Die granuläre Stärke wird erfindungsgemäß in Form von Partikeln eingesetzt, wobei diese Partikel in ihrer Form den ursprünglichen Stärkekörnern entsprechen oder Agglomerate davon sind. Typische Grössen der Stärkekörner im nicht gequollenen Zustand sind: 5 - 100 µm bei Kartoffelstärke, 5 - 30 µm bei Maisstärke, 1 - 45 µm bei Weizensstärke, 4 - 35 µm bei Tapiocastärke, 1 - 30µm bei Reisstärke. Bei einer teilweisen Destrukturierung können die ursprünglichen Stärkekörner bezüglich Geometrie und Grösse verändert worden sein, insbesondere nimmt die Grösse bei der Destrukturierung deutlich zu. Als granuläre Stärke können auch Mischungen von verschiedenen granulären Stärken eingesetzt werden.

Bevorzugt ist der Anteil der granulären Stärke am gesamten Stärkeanteil der Mischung in Gew.-% > 60, besonders bevorzugt > 70, noch bevorzugter > 75, noch bevorzugter > 80, noch bevorzugter > 85, ganz besonders bevorzugt > 90.

### Wasser

Wasser ist für die Einstellung der Viskosität der Gießmasse und für die Verfestigung der Weichkapseln nach der Umformung der Gießmasse zu einem Film von Bedeutung. Je höher der Wassergehalt ist, umso einfacher ist das Gießen, umso schneller erfolgt die Verfestigung und umso geringer ist die dafür notwendige Temperaturerhöhung. Andererseits reduziert ein hoher Wassergehalt die Festigkeit der Weichkapseln und es werden längere Trocknungszeiten benötigt, da dann mehr Wasser aus dem Film bzw. aus der Weichkapsel entfernt werden muss.

Die obere Grenze für den Wassergehalt der Gießmasse in Gew.-% beträgt bevorzugt 90, besonders bevorzugt 80, noch bevorzugter 70, noch bevorzugter 60, noch bevorzugter 50, noch bevorzugter 45, ganz besonders bevorzugt 40, während die untere Grenze des Wassergehalts der Gießmasse in Gew.-% vorzugsweise 15, besonders bevorzugt 20, noch bevorzugter 25, noch bevorzugter 30, ganz besonders bevorzugt 33 beträgt. Mit zunehmendem Wassergehalt wird die Verfestigung erleichtert, z.B. bei tieferen Temperaturen ermöglicht und/oder beschleunigt, jedoch wird die Festigkeit des verfestigten Films dabei reduziert und die Wassermenge, die nach der Verfestigung wieder entfernt werden muss vergrössert.

### Weichmacher

Als Weichmacher kommen grundsätzlich alle im Stand der Technik aufgeführten Weichmacher für Stärke in Frage. Ein tiefer Weichmachergehalt führt zu Versprödung der Weichkapseln bei tiefen Luftfeuchtigkeiten, während ein hoher Weichmachergehalt zu schlechten Eigenschaften bei hoher Luftfeuchtigkeit führt.

Weichmacher können einzeln oder in Mischungen von verschiedenen Weichmachern eingesetzt werden. Vorzugsweise werden Polyole eingesetzt wie beispielsweise Glycerin, Sorbitol, Maltitol, Erythritol, Xylitol, Mannitol, Galactitol, Tagatose, Lactitol, Maltulose, Isomalt, Maltol usw., aber auch verschiedene Zucker wie Sucrose, Maltose, Trehalose, Lactose, Lactulose, Galactose, Fructose usw. sowie Mono- und Oligosaccharide. Glycerin ist als Weichmacher besonders bevorzugt. Sucrose hat neben seiner Eigenschaft als Weichmacher weiterhin den Vorteil, die Sauerstoffbarriereeigenschaften der Weichkapsel zu verbessern. Wasser ist ebenfalls ein Weichmacher für Stärke, wird hier jedoch nicht zu den Weichmachern gerechnet und separat berücksichtigt.

Die obere Grenze für den Weichmachergehalt in Gew.-% der trockenen Mischung beträgt vorzugsweise 70, besonders bevorzugt 60, noch bevorzugter 55, noch bevorzugter 50, noch bevorzugter 46, ganz besonders bevorzugt 42, während die untere Grenze in Gew.-% vorzugsweise 15, besonders bevorzugt 20, noch bevorzugter 25, noch bevorzugter 28, noch bevorzugter 31 noch bevorzugter 32,5, ganz besonders bevorzugt 33,5 beträgt.

In einer bevorzugten Ausführung werden Weichmacher mit einer maximalen Schmelztemperatur des wasserfreien Weichmachers von 150°C, vorzugsweise 125°C, besonders bevorzugt 110°C, noch bevorzugter 95°C, ganz besonders bevorzugt 70°C eingesetzt. Der Anteil der Weichmacher am Gesamtweichmachergehalt, welcher diese Bedingung erfüllt, beträgt in Gew.-% > 50, vorzugsweise > 70, besonders bevorzugt > 80, ganz besonders bevorzugt > 90.

### Optionale Bestandteile der Stärkemischung

### Kurzkettige Amylose

Die Stärkemischung kann einen Anteil an kurzkettiger Amylose aufweisen. Diese kurzkettige Amylose kann beispielsweise durch Einwirkung von Enzymen auf die granuläre Stärke in der granulären Stärke erhalten werden oder durch Besprühen der granulären Stärke mit gelöster kurzkettiger Amylose auf die granuläre Stärke aufgebracht werden. Diese kurzkettige Amylose kann zusammen mit mindestens einer der Stärken, die für die Herstellung des Films eingesetzt werden, zugeführt werden, oder sie kann der Mischung auch separat zugeführt werden, beispielsweise in Form einer Lösung von kurzkettiger Stärke oder in Form von sprühgetrockneter kurzkettiger Stärke, wobei die sprühgetrocknete kurzkettige Stärke noch andere sprühgetrocknete Stärken als Mischung aufweisen kann. Bevorzugt liegt die kurzkettige Amylose in und/oder auf der granulären Stärke in nicht-kristalliner Form vor. Kurzkettige Amylose (Short Chain Amylose) besteht aus substanziell unverzweigten Amylosen und wird in einer bevorzugten Ausführung eingesetzt. Der Verzweigunsgrad (Anzahl Verzweigungen pro Monomereinheit) der kurzkettigen Amylosen ist < 0,01, vorzugsweise < 0,005, besonders bevorzugt < 0,003, noch bevorzugter < 0,001, noch bevorzugter < 0,0007, noch bevorzugter < 0,0004, ganz besonders bevorzugt < 0,0001. Idealerweise hat die kurzkettige Amylose einen Verzweigungsgrad von 0 oder nahe null, beispielsweise wenn sie durch vollständige Entzweigung (beispielsweise mittels Pullulanase) erhalten wird. Mit abnehmendem Verzweigungsgrad nimmt die Kristallisierbarkeit der kurzkettigen Amylose zu und damit auch die Netzwerkbildung (unter Heterokristallisation mit längeren Stärke Makromolekülen), welche die kurzkettige Amylose bewirkt. Mit zunehmender Netzwerkbildung werden verbesserte Eigenschaften der erfindungsgemäßen Weichkapseln erhalten, insbesondere höhere E-Moduli bei hohen Luftfeuchtigkeiten, wodurch die Weichkapseln in einem breiten Bereich von Klimazonen mit unterschiedlichen Luftfeuchtigkeiten einsetzbar sind.

Kurzkettige Amylose weist einen mittleren Polymerisationsgrad (DPn: Zahlenmittel) von > 8 und < 500 auf. Erfindungsgemäß beträgt er vorzugsweise < 300, besonders bevorzugt < 100, noch bevorzugter < 70, noch bevorzugter < 50, ganz besonders bevorzugt < 30 auf. Weiterhin ist erfindungsgemäß bevorzugt, dass der mittlere Polymerisationsgrad > 10 beträgt, besonders bevorzugt > 12, noch bevorzugter > 14, ganz besonders bevorzugt > 15. Mit abnehmendem DPn wird die Transparenz der Weichkapseln verbessert, da die Heterokristallite bestehend aus kurzkettiger Amylose und längeren Stärke Makromolekülen mit abnehmendem DPn der kurzkettigen Amylose kleiner werden, wodurch die Lichtstreuung reduziert wird. Bei zu tiefem DPn ist Kristallisation nicht mehr möglich.

Kurzkettige Amylose kann beispielsweise durch Polymerisation von Glucose synthetisch oder durch die Wirkung von Enzymen (beispielsweise α-Amylase, β-Amylase, Isoamylase, Pullulanase) aus Stärke erhalten werden.

Bevorzugt ist der Anteil der kurzkettigen Amylose am gesamten Stärkeanteil der Mischung in Gew.-% < 15, besonders bevorzugt < 10, noch bevorzugter < 7.5, noch bevorzugter < 5, noch bevorzugter < 3, ganz besonders bevorzugt = 0.

### Verdickungsmittel

Der Stärke enthaltenden Mischung kann ein Verdickungsmittel zugesetzt werden, um die Viskosität der Mischung auf einen gewünschten Wert einzustellen, es ermöglicht also eine Optimierung der Viskosität der Mischung beim Gießen. Ausserdem werden Verdickungsmittel vorteilhaft eingesetzt, um die Verbindungen zwischen den destrukturierten Stärkepartikeln in den verfestigten Weichkapseln hinsichtlich eines beschleunigten Zerfallsverhaltens in wässrigem Medium zu schwächen. Das Verdickungsmittel kann zum Zeitpunkt der Umformung der Mischung in Form von Partikeln, in gequollener Form oder in gelöster Form vorliegen.

Als Verdickungsmittel kommen grundsätzlich alle hydrophilen Substanzen und Mischungen davon in Frage, welche die Viskosität erhöhen, insbesondere hydrophile Polymere und davon vorzugsweise solche aus pflanzlichen Quellen. Beispiele sind Hydrokolliode und Gummis wie Galactomannane, wie Guar-Gummi oder Johannisbrotkernmehl; Cellulosederivate; Pectine, insbesondere Rhamnogalakturonane und Protopektine; Dextrane; Xanthan; Zymosan; Hydrokolloide aus Meeresalgen, wie Alginate, Agar-Agar, Agarose, Carrageen und Carrageenane; Furcellaran; Hydrokolloide aus Flechten, wie Lichenine und Isolichenine, oder Hydrokolloide als Exsudate aus Hölzern, wie Tragant (Astragalus Gummi), Karaya-Gummi, Gummi arabicum, Kutira-Gummi; Inulin; Latex; Chitin; Chitosan; Gellan; Kollagen; Gelatine; Casein. Gelöste Stärke kann für dieselbe Funktionalität wie die Verdickungsmittel eingesetzt werden, wird aber nicht zu den Verdickungsmitteln gerechnet und separat behandelt.

Einige dieser Verdickungsmittel wie beispielsweise Gelatine, Carrageenan, Gellan und Pectin sind auch als Geliermittel bekannt, wobei sie allerdings beim Abkühlen gelieren und nicht beim Erhitzen. Sie leisten bei der Verfestigung der erfindungsgemäßen Gießmischung bei der Temperaturerhöhung keinen Beitrag zur Gelierung und werden auch nicht hierfür eingesetzt.

In einer bevorzugten Ausführungsform beträgt der maximale Anteil an Verdickungsmittel in Gew.-%, bezogen auf die trockene Rezeptur, nach Abzug des Weichmachers 50, noch bevorzugter 40, noch bevorzugter 30, noch bevorzugter 20, noch bevorzugter 10, noch bevorzugter 5, noch bevorzugter 2,5, ganz besonders bevorzugt 1.

In einer weiteren bevorzugten Ausführungsform beträgt der maximale Anteil an Carrageen und Carrageenanen in Gew.-%, bezogen auf die trockene Rezeptur, nach Abzug des Weichmachers 10, noch bevorzugter 7,5, noch bevorzugter 5, noch bevorzugter 3, noch bevorzugter 2, noch bevorzugter 1, noch bevorzugter 0,5, ganz besonders bevorzugt 0. Aufgrund der hohen Rohstoffkosten und des Verdachts auf Kanzerogenität wird der Anteil an Carrageen und Carrageenanen möglichst tief gehalten.

In einer weiteren bevorzugten Ausführungsform beträgt der maximale Anteil an Gelatine in Gew.-%, bezogen auf die trockene Rezeptur, nach Abzug des Weichmachers 10, noch bevorzugter 7,5, noch bevorzugter 5, noch bevorzugter 3, noch bevorzugter 2, noch bevorzugter 1, noch bevorzugter 0,5, ganz besonders bevorzugt 0. Aufgrund der generellen Gelatine-Problematik wird der Anteil an Gelatine möglichst tief gehalten.

In einer weiteren bevorzugten Ausführungsform beträgt der maximale Anteil an Gellan in Gew.-%, bezogen auf die trockene Rezeptur, nach Abzug des Weichmachers 5, noch bevorzugter 2,5, noch bevorzugter 2, noch bevorzugter 1,5, noch bevorzugter 1, noch bevorzugter 0,5, noch bevorzugter 0,2, ganz besonders bevorzugt 0. Aufgrund der hohen Rohstoffkosten wird der Anteil an Gellan möglichst tief gehalten.

In einer weiteren bevorzugten Ausführungsform beträgt der maximale Anteil an Pektin in Gew.-%, bezogen auf die trockene Rezeptur, nach Abzug des Weichmachers 5, noch bevorzugter 2,5, noch bevorzugter 2, noch bevorzugter 1,5, noch bevorzugter 1, noch bevorzugter 0,5, noch bevorzugter 0,2, ganz besonders bevorzugt 0. Aufgrund der hohen Rohstoffkosten und der problematischen Verarbeitbarkeit wird der Anteil an Pektin möglichst tief gehalten.

In einer weiteren bevorzugten Ausführungsform beträgt der maximale Anteil an Cellulosederivaten in Gew.-%, bezogen auf die trockene Rezeptur, nach Abzug des Weichmachers 15, noch bevorzugter 10, noch bevorzugter 5, noch bevorzugter 2,5, noch bevorzugter 1, noch bevorzugter 0,5, ganz besonders bevorzugt 0. Aufgrund der hohen Rohstoffkosten und der Entmischung bzw. dem Ausfallen von Cellulosederivaten aus der Stärkemischung bei erhöhten Temperaturen wird der Anteil an Cellulosederivaten möglichst tief gehalten.

### Gelöste Stärke

Gelöste Stärke kann ebenso wie die zuvor erwähnten Verdickungsmittel eingesetzt werden, um die Viskosität der Mischung zu erhöhen und um die Verbindung zwischen den Stärke Partikeln zu modifizieren. Ihr Einsatz ist optional, da die gewünschte Erhöhung der Viskosität auf die für das Gießen geeignete Viskosität auch durch eine geeignete Erhöhung der Temperatur der Gießmischung erhalten werden kann, wobei die granuläre Stärke infolge Quellung die Viskosität erhöht. Da dann jedoch die Temperatur der Gießmischung genau eingestellt und kontrolliert werden muss, ist die Verfahrensweise mit Einsatz von gelöster Stärke (oder einem Verdickungsmittel) einfacher und daher bevorzugt.

Betreffend die gelöste Stärke gelten dieselben Aussagen zu geeigneten Stärken und bevorzugten Typen wie betreffend die Stärke allgemein. Jedoch kann die gelöste Stärke auch ein tieferes Molekulargewicht aufweisen als für die Stärke allgemein bevorzugt ist. Ausserdem werden für die gelöste Stärke noch bevorzugt stark retrogradationsstabilisierte Stärken eingesetzt, bsw. hochsubstituierte Stärken oder hochverzweigte Dextrine, wodurch der Zerfall der Weichkapsel in wässrigem Medium beschleunigt werden kann.

Die gelöste Stärke unterscheidet sich von der granulären Stärke in ihrem Zustand in der Gießmischung, wo sie in gelöster Form oder in vorwiegend destrukturierter Form vorliegt, während die granuläre Stärke zu diesem Zeitpunkt noch vorwiegend nicht destrukturiert vorliegt.

Gelöste Stärke kann beispielsweise durch Lösen aus amorpher, extrudierter Stärke erhalten werden oder sie kann aus pregelatinisierter Stärke erhalten werden. Erfindungsgemäß wird unter dem Begriff "gelöste Stärke" auch pregelatinisierte Stärke verstanden (wie beispielsweise walzengetrocknete pregelatinisierte Stärke), selbst wenn diese in ungelöster Form oder nur teilweise gelöst vorliegt. Pregelatinisierte Stärke ist bevorzugt mindestens bis zur Stufe 2.3 destrukturiert, noch bevorzugter mindestens bis zur Stufe 2.4, noch bevorzugter mindestens bis zur Stufe 3.1, noch bevorzugter mindestens bis zur Stufe 3.3.

In einer bevorzugten Ausführung ist gelöste Stärke spätestens zum Zeitpunkt zu dem die Mischung zu einem Film umgeformt wird, mindestens bis zur Stufe 2.3 destrukturiert, noch bevorzugter mindestens bis zur Stufe 2.4, noch bevorzugter mindestens bis zur Stufe 3.1, noch bevorzugter mindestens bis zur Stufe 3.3, noch bevorzugter mindestens bis zur Stufe 3.5, noch bevorzugter mindestens bis zur Stufe 3.6, besonders bevorzugt mindestens bis zur Stufe 4.1, ganz besonders bevorzugt bis zur Stufe 4.2.

Weiterhin ist erfindungsgemäß bevorzugt, dass die obere Grenze für den Anteil der gelösten Stärke bezogen auf die wasserfreie Mischung in Gew.-% 30 beträgt, besonders bevorzugt 25, noch bevorzugter 20, noch bevorzugter 15, noch bevorzugter 10, ganz besonders bevorzugt 5.

### Weitere Bestandteile (Zusatz- und Hilfsstoffe)

Weitere Bestandteile der Stärkemischung können Farbstoffe und Pigmente sein sowie Füllstoffe, mineralische Füllstoffe wie beispielsweise Talk, oder modifizierende Stoffe wie Polyethlyenglycole oder Zerfallshilfen wie beispielsweise Carbonate oder Hydrogencarbonate oder Additive wie beispielsweise Konservierungsmittel, Antioxidantien oder Emulgatoren wie beispielsweise Lecithine, Mono-, Di- und Triglyceride von Fettsäuren, Polyglycerinexter, Polyethylenester oder Zuckerester. Grundsätzlich können alle Zusatzstoffe, welche bei Gelatineweichkapselhüllen eingesetzt werden, erfindungsgemäß ebenfalls eingesetzt werden, insbesondere Zusatzstoffe, welche eingesetzt werden um die Weichkapselhülle auf den Inhaltsstoff anzupassen (Formulierungshilfsstoffe).

### Formgebung und Verfestigung

Die Stärke in Form von Partikeln von granulärer Stärke wird während und/oder nach der Umformung der Mischung zu einem Film durch eine Temperaturerhöhung destrukturiert, wodurch eine rasche Verfestigung der Gießmischung zu einem festen Film erhalten wird. Bevorzugt findet die Temperaturerhöhung nach der Umformung der Mischung zu einem Film statt, insbesondere unmittelbar nach der Umformung der Mischung zu einem Film. Gegebenenfalls beträgt die Temperaturerhöhung während der Umformung höchstens 50%, vorzugsweise höchstens 40%, noch bevorzugter höchstens 30%, noch bevorzugter höchstens 20%, am bevorzugtesten höchstens 10% der gesamten Temperaturerhöhung der Gießmasse auf die Verfestigungstemperatur.

In einer bevorzugten Ausführungsform kann die Mischung, enthaltend Stärke, unter einem Druck von weniger als 5 bar (0,5 MPa), besonders bevorzugt weniger als 4 bar (0,4 MPa), noch bevorzugter weniger als 3 bar (0,3 MPa), noch bevorzugter weniger als 2 bar (0,2 MPa), ganz besonders bevorzugt weniger als 1 bar (0,1 MPa) umgeformt werden. Bei solchen Drücken ist der Druckaufbau einfach und die benötigten Geräte sind einfach und günstig. In noch einer weiteren bevorzugten Ausführungsform kann die Mischung enthaltend Stärke unter einem Druck von weniger als 0,7 bar (0,07 MPa), besonders bevorzugt weniger als 0,6 (0,06 MPa) bar, noch bevorzugter weniger als 0,5 bar (0,05 MPa), noch bevorzugter weniger als 0,4 bar (0,04 MPa), noch bevorzugter weniger als 0,3 bar (0,03 MPa), ganz besonders bevorzugt weniger als 0,2 bar (0,02 MPa) umgeformt werden. In der bevorzugtesten Ausführung wird die Mischung praktisch drucklos umgeformt, d.h. die Mischung fliesst infolge ihres Eigengewichts durch die Formgebungseinheit, welche beispielsweise eine Spreader Box ist, welche bereits beim Gießen von Gelatine standardmässig eingesetzt wird.

Grundsätzlich kann die Viskosität der Gießmischung beispielsweise mit Verdickungsmitteln auch so hoch eingestellt werden, dass Drücke von weit oberhalb 5 bar (0,5MPa) für die Umformung der Gießmischung zum Film benötigt werden.

Bevorzugt liegt die obere Grenze für die dynamische Viskosität der Mischung vor oder bei der Umformung (d.h. die Viskosität bei der entsprechenden Temperatur) in Pas bei 3000, besonders bevorzugt 1000, noch bevorzugter 500, noch bevorzugter 300, noch bevorzugter 200, noch bevorzugter 150, noch bevorzugter 120, noch bevorzugter 100, noch bevorzugter 70, ganz besonders bevorzugt 50. Weiterhin ist bevorzugt, dass die untere Grenze für die dynamische Viskosität der Mischung vor oder bei der Umformung in Pas bei 0,01, besonders bevorzugt 0,05, noch bevorzugter 0,1, noch bevorzugter 0,5, ganz besonders bevorzugt 1 liegt. Die Viskositäten beziehen sich auf eine Schergeschwindigkeit von 1,1/s. Hohe Viskositäten korrelieren mit hohen notwendigen Drücken, sodass die Vorteile der tieferen Viskositäten den Vorteilen der tieferen Drücken entsprechen. Da eine Vielzahl von Möglichkeiten bestehen, Mischungen mit einem weiten Bereich von Viskositäten umzuformen, decken die in Frage kommenden Viskositäten einen entsprechend weiten Bereich ab. Bei einer Viskosität unterhalb von etwa 300 Pas sind drucklose Gießverfahren (unter Eigengewicht der Mischung) mittels der für das Gelatine-Gießverfahren typischen Spreader Box möglich. Die unteren Grenzen sind dadurch gegeben, dass bei sehr tiefen Viskositäten die Formgebung und insbesondere die Einstellung der Dicke eines gegossenen Films zunehmend schwierig wird (Wegfliessen der Mischung).

Bevorzugt liegt die obere Grenze für die Temperatur in °C mit der die Mischung, enthaltend Stärke, umgeformt wird bei 90, besonders bevorzugt bei 80, noch bevorzugter 70, noch bevorzugter 65, noch bevorzugter 60, noch bevorzugter 55, ganz besonders bevorzugt 50. Weiterhin liegt in einer bevorzugten Ausführungsform die untere Grenze für die Temperatur in °C mit der die Mischung enthaltend Stärke umgeformt wird bei -20, besonders bevorzugt bei -10, noch bevorzugter 0, noch bevorzugter 10, noch bevorzugter 20, noch bevorzugter 30, noch bevorzugter 35, noch bevorzugter 40, ganz besonders bevorzugt 45.

Ausgehend von der Temperatur der Giessmaße vor der Umformung, d.h. von der Temperatur der Gießmasse in der Spreader Box, wird die Temperatur der Stärkemischung erhöht, um diese zu verfestigen. Bevorzugt liegt die untere Grenze für die Temperaturerhöhung der Stärkemischung in °C zur Auslösung der Verfestigung bei 10, besonders bevorzugt 15, noch bevorzugter 20, noch bevorzugter 25, noch bevorzugter 30, noch bevorzugter 35, ganz besonders bevorzugt 40. Weiterhin liegt in einer bevorzugten Ausführungsform die obere Grenze der Temperaturerhöhung in °C bei 130, noch bevorzugter 110, noch bevorzugter 90, ganz besonders bevorzugt 70. Mit zunehmender Temperaturerhöhung wird die Verfestigung beschleunigt und werden bessere mechanische Eigenschaften erhalten, da die Stärkepartikel besser aneinander gebunden werden. Die obere Grenze ist durch die mit zunehmender Temperatur auftretende bzw. zunehmende Blasenbildung gegeben.

Bevorzugt wird der Wassergehalt nach der Umformung der Gießmasse während der Verfestigung des Produktes in etwa konstant gehalten, insbesondere bis der Film (bei Raumtemperatur) einen E-Modul in MPa von mindestens 0,001, vorzugsweise 0,003, besonders bevorzugt 0,005, noch bevorzugter 0,007, noch bevorzugter 0,009, ganz besonders bevorzugt 0,01 erreicht hat. Während der Verfestigung wird der Wassergehalt bevorzugt höchstens um 25 Gew.-%, besonders bevorzugt um höchstens 20 Gew.-%, noch bevorzugter um höchstens 15 Gew.-%, noch bevorzugter um höchstens 10 Gew.-%, noch bevorzugter um höchstens 7 Gew.-%, noch bevorzugter um höchstens 5 Gew.-%, ganz besonders bevorzugt um höchstens 3 Gew.-% reduziert (zur Verdeutlichung: liegt der Wassergehalt nach der Umformung der Gießmasse zu einem Film bei 40%, so liegt der nach einer Reduktion von 3% bei 37%). Die Konstanz des Wassergehaltes während der Verfestigung des Films erleichtert die Verfestigung, eine zu starke Reduktion des Wassergehalts in dieser Phase führt zu unvollständiger Verfestigung des Films und somit zu ungenügenden mechanischen Eigenschaften, insbesondere neigt der Film bei der weiteren Verarbeitung dann zu Rissbildung.

### Weichkapseln

Erfindungsgemäße Weichkapseln auf Stärkebasis umfassen bevorzugt:
a) > 40 Gew.-% der trockenen Weichkapsel, nach Abzug des Weichmachers, Stärke,
b) 15 - 70 Gew.-% der trockenen Weichkapsel Weichmacher, und
c) 0,1 - 50 Gew.-% der gesamten Weichkapsel Wasser,
d) gegebenenfalls maximal 50 Gew.-% der trockenen Weichkapsel, nach Abzug des Weichmachers, eines Verdickungsmittels, und
e) gegebenenfalls übliche Zusatz- und Hilfsstoffe,
wobei die Weichkapsel miteinander verbundene Stärkepartikel aufweist, insbesondere miteinander verbundene Partikel destrukturierter Stärke.
Bevorzugt bilden die miteinander verbundenen Stärkepartikel eine Matrix, wobei in dieser Matrix gegebenenfalls weitere Phasen eingeschlossen sind. Bevorzugt beträgt der Anteil der weiteren Phasen in Gew.-% < 30, noch bevorzugter < 20, noch bevorzugter < 10, noch bevorzugter < 5, noch bevorzugter < 2,5, am bevorzugtesten < 1,5.

Diese Stärkepartikel in den Weichkapseln sind destrukturierte Stärkepartikel, die aus der granulären Stärke bei der Gelierung der Gießmischung zum Film entstanden sind, wobei gegebenenfalls noch destrukturierte Stärkepartikel vorliegen, die bereits vor der Gelierung der Gießmischung in diesem Zustand vorlagen und von der gelösten Stärke stammen (wobei ihr Destrukturierungsgrad bevorzugt mindestens demjenigen der granulären Stärke entspricht). Bevorzugt existieren die Stärkepartikel der granulären Stärke noch als individuelle Stärkepartikel, bevorzugt mit einem mittleren Durchmesser von mindestens 2 µm, noch bevorzugter von mindestens 4µm, noch bevorzugter von mindestens 6µm. Die aus granulärer Stärke entstandenen Stärkepartikel sind bevorzugt mindestens bis zur Stufe 2.1 destrukturiert, besonders bevorzugt bis zur Stufe 2.2, noch bevorzugter bis zur Stufe 2.3, noch bevorzugter bis zur Stufe 2.4 ganz besonders bevorzugt bis zur Stufe 3.1. Mit zunehmender Destrukturierung werden die Handhabung des frischen Films die mechanischen und optischen Eigenschaften der frischen und getrockneten Weichkapsel verbessert. Andererseits sind die Stärkepartikel bevorzugt höchstens bis zur Stufe 4.1 destrukturiert, besonders bevorzugt bis zur Stufe 3.6, noch bevorzugter bis zur Stufe 3.5, noch bevorzugter bis zur Stufe 3.4, noch bevorzugter bis zur Stufe 3.3, ganz besonders bevorzugt bis zur Stufe 3.2. Um eine sehr hohe Destrukturierung zu erreichen, sind sehr hohe Temperaturen bei der Verfestigung notwendig, was verfahrenstechnisch aufwändig zu kontrollieren ist, insbesondere die Kontrolle des Wassergehalts sowie die Entstehung von unerwünschten Luftblasen. Ausserdem nimmt bei sehr hoher Destrukturierung, wenn die Stärke Körner zunehmend zerfallen, der positive Beitrag der Stärke Partikel zu den mechanischen Eigenschaften des frischen Films und der Weichkapseln ab.

Die granuläre Stärke liegt zum Zeitpunkt der Umformung der Mischung zu einem Film als fester, höchstens teilweise gequollener Partikel vor. Im verfestigten Film liegt diese Stärke in Form von stark gequollenen, destrukturierten Stärkepartikeln vor, die miteinander verbunden sind, entweder direkt durch Kopplung von Oberflächen solcher Partikel oder indirekt über eine Zwischenschicht, wobei diese Zwischenschicht gegebenenfalls ein Bindemittel und/oder Stärke, insbesondere gelöste Stärke aufweisen kann. Das Verhältnis der mittleren Dicke der Zwischenschicht dividiert durch den mittleren Durchmesser der gequollenen Partikel beträgt bevorzugt < 0,4, besonders bevorzugt < 0,2, noch bevorzugter 0,15, noch bevorzugter < 0, 1, noch bevorzugter < 0,05. D.h. bevorzugt sind die Partikel dicht gepackt, am bevorzugtesten berühren die Partikel einander in einer dichten, insbesondere einer dichtesten Packung (d.h. eine Packung ohne Zwischenräume).

Die Verbindung zwischen den Stärkepartikeln kann gegebenenfalls durch die gelöste Stärke zwischen den Partikeln oder durch ein anderes Bindemittel verbessert werden, aber auch ohne diese Massnahme wird eine ausreichende Verbindung erhalten. Der Aufbau des Stärkefilms bzw. der Weichkapsel als ein dichtes Agglomerat von Partikeln kommt deutlich zum Ausdruck, wenn der Film bzw. die Weichkapsel in Wasser gelegt und mit einem Magnetrührer bewegt wird, z.B. bei Raumtemperatur oder bei 70°C. Die Weichkapsel zerfällt, gegebenenfalls (bei Raumtemperatur) unter Einwirkung von geringfügigem Zerreiben, zunächst in eine feine gleichmässige Paste. Wird diese Masse weiter mit Wasser verdünnt, können daraus wieder individuelle Stärkepartikel gewonnen werden, die unter dem Lichtmikroskop aufgrund ihrer Form als gequollene Partikel der destrukturierten Stärke identifiziert werden können. Aus destrukturierten Stärkekörnern, die aus der Weichkapsel zurück gewonnen werden können, lässt sich sogar der Ursprung bzw. Typ der eingesetzten Stärke ermitteln, da unterschiedliche Stärken unterschiedliche Kornformen und Korngrössenverteilungen aufweisen. Um die Partikel im Mikroskop deutlich zu machen, werden sie vorteilhaft mit einer Iod-Lösung (Lugolsche Lösung) angefärbt. Eine andere Möglichkeit die ursprünglichen Stärkepartikel wieder sichtbar zu machen, besteht darin, dass statt der Anfärbung ein Tropfen der mit Wasser verdünnten Masse auf einen Objektträger gegeben wird. Nachdem das Wasser verdunstet ist, können die Stärkepartikel im Lichtmikroskop identifiziert werden. Aufgrund der Schrumpfung der gequollenen Stärkepartikel beim Trocknen weisen diese Partikel charakteristische Deformationen und gegebenenfalls Risse auf.

Das bevorzugte Gewichtsmittel der Molekulargewichtsverteilung M_{w}2 der enthaltenen Stärke liegt, ebenso wie das bevorzugte Gewichtsmittel der Molekulargewichtsverteilung M_{w}1 der Stärke in der Stärkegießmischung, mindestens bei 500.000 g/mol, besonders bevorzugt mindestens 1.000.000 g/mol, noch bevorzugter mindestens 2.500.000 g/mol, noch bevorzugter mindestens 3.000.000 g/mol, noch bevorzugter mindestens 4.000.000 g/mol, noch bevorzugter mindestens 5.000.000 g/mol, noch bevorzugter mindestens 7.000.000 g/mol, ganz besonders bevorzugt mindestens 10.000.000 g/mol.

Hinsichtlich der Bestandteile der Weichkapsel gelten bis auf den Wassergehalt die Aussagen zur im Verfahren verwendeten Gießmischung. Die obere Grenze für den Wassergehalt der erfindungsgemäßen Weichkapsel in Gew.-% liegt vorzugsweise bei 40, besonders bevorzugt 30, noch bevorzugter 25, noch bevorzugter 20, ganz besonders bevorzugt 17, während die untere Grenze des Wassergehalts der Weichkapsel in Gew.-% vorzugsweise 1 beträgt, besonders bevorzugt 3, noch bevorzugter 5, ganz besonders bevorzugt 7. Mit höher werdendem Wassergehalt verliert die Weichkapsel ihre mechanischen Eigenschaften, und wird insbesondere zu weich. Mit tiefer werdendem Wassergehalt wird die Weichkapsel zu hart.

### Unlösliche Bestandteile des Films bzw. der Weichkapsel

Die hergestellten Filme bzw. Weichkapselhüllen bestehen aus Partikeln von Stärke, die in einer bevorzugten Ausführung dicht gepackt sind, woraus sich vorteilhafte Eigenschaften für die Verarbeitung des Films und für die Eigenschaften des fertigen Films ergeben. Diese Partikeln von Stärke können z.B. durch Auflösen des Films bei 70°C während 30 min von den löslichen Bestandteilen (diese sind insbesondere Weichmacher, lösliche Stärke, gegebenenfalls Verdickungsmittel) separiert werden und ihr quantitativer Anteil im Film kann somit gemessen werden.

### Wiedergewinnungsverfahren Nr. 1

In einer bevorzugten Ausführung beträgt der minimale Anteil in Gew.-% der Stärke in der Weichkapselhülle, der nach Auflösen der Weichkapsel bei 70°C während 30 min zurück gewonnen werden kann, 30, vorzugsweise 40, noch bevorzugter 50, noch bevorzugter 55, noch bevorzugter 60, noch bevorzugter 65, ganz besonders bevorzugt 70%.

### Wiedergewinnungsverfahren Nr. 2

In einer weiteren bevorzugten Ausführung wird der Anteil der Masse bestimmt, die nach Auflösen der Weichkapselhülle bei 70°C während 30 min zurück gewonnen werden kann, und auf die Masse des trockenen Films bezogen. Die Bestimmung nach dieser Definition ist einfacher als jene gemäß Wiedergewinnungsverfahren Nr. 1, weil sie auch dann angewendet werden kann, wenn die Zusammensetzung der Weichkapselhülle nicht genau bekannt ist. Der minimale Anteil in Gew.-% der Masse, die zurück gewonnen werden kann, liegt bei 25, vorzugsweise 35, noch bevorzugter 40, noch bevorzugter 45, ganz besonders bevorzugt 50.

### Vorteile des erfindungsgemäßen Verfahrens und der erfindungsgemässen Weichkapseln

Erfindungsgemäße Gießmischungen sind einfach herzustellen (Erfindung: einfacher Mischvorgang; Gelatine: aufwändige Gelherstellung; Thermoplastische Stärke (TPS): Herstellung eines Granulats, welches wegen seiner Klebrigkeit schwierig zu handeln ist und verklumpt). Der Gießprozess an sich ist jedoch vorteilhaft ähnlich wie bei Gelatine, d. h. druckloses Gießen unter Eigengewicht ist möglich, sodass die Umstellung vom Gelatinegießen auf das erfindungsgemäße Verfahren möglich ist, aber die Verfestigung erfolgt durch Temperaturerhöhung und nicht durch Temperaturerniedrigung. Durch die schnelle Verfestigung (Gelierung) nach dem Gießen, werden hohe und kompetitive Produktionsraten erhalten. Der Film ist wie ein Gelatinefilm isotrop, d.h. seine Eigenschaften sind nicht richtungsabhängig.

Das wesentliche Merkmal der Stärkemischung, welche im erfindungsgemäßen Verfahren verwendet wird, besteht darin, dass diese Mischung Stärke in Form von Partikeln aufweist, d.h. die Mischung ist eine Dispersion der Partikeln in wässrigem Medium. Diese Mischung ist über längere Zeit stabil.

Die Verkapselung erfordert eine Dehnbarkeit des Films von mindestens 100%. Beim Schliessen der Weichkapselhülle sollte der Film mit sich selbst verschweissbar sein und die Schweissnaht sollte sofort belastbar sein. Die frische Kapsel sollte stabil genug sein für die nächsten Verarbeitungsschritte (Transport weg vom "Rotary Die" und Reinigung im Tumbler (Entfernung des Rotary Die Öls). Diese Bedingungen werden durch das erfindungsgemäße Verfahren erfüllt.

Rohstoffkosten und Verfahrenskosten für die erfindungsgemäße Kapselherstellung sind geringer als bei der Herstellung von Gelantineweichkapseln.

Der Film zur Herstellung von Weichkapseln kann nach der Verfestigung einen E-Modul mindestens 0,009 MPa und eine Bruchdehnung von mindestens 100 % aufweisen. E-Modul und Bruchdehnung werden bei Raumtemperatur gemessen, unmittelbar nach der Verfestigung, d.h. höchstens einige Minuten nach der Umformung der Mischung zu einem Film, wobei der Wassergehalt dem Wassergehalt nach der Verfestigung des Films entspricht. Wird die Verfestigung beispielsweise auf einer rotierenden Trommel erreicht, dann werden E-Modul und Bruchdehnung des Films gemessen, nachdem der Film die Trommel verlassen hat und der Wassergehalt bei der Messung entspricht dem Wassergehalt des Films zu diesem Zeitpunkt. Erst mit genügend grossem E-Modul und ausreichender Bruchdehnung nach der Verfestigung wird die Handhabbarkeit des Films möglich, da für die Weiterverarbeitbarkeit der verfestigte Film mechanisch beansprucht wird. Die Eigenschaften des frischen Films sind hierzu mehr als ausreichend, sodass auch hohe Produktionsraten gefahren werden können.

Erfindungsgemäße Weichkapseln haben ebenfalls gute mechanische Eigenschaften, insbesondere hohe Elastizität und hohes Dehnvermögen. Die Weichkapseln sind aus miteinander verbundenen, dicht gepackten, individuellen Stärkepartikeln aufgebaut. Diese Stärkepartikel liegen in einem gequollenen Zustand und bevorzugt in einer dichten Packung vor. Die Weichkapseln sind ausserdem kompakt und frei von Luftblasen. Bisher wurde von der Fachwelt vorausgesetzt, dass für brauchbare Weichkapseln die Stärke im Extruder plastifiziert werden muss, wobei die Individualität der eingesetzten Stärkepartikel, typischerweise granuläre Stärke, vollständig verloren geht.

Noch überraschender ist in Anbetracht der partikulären Struktur, wobei man zunächst erwarten müsste, dass die Verbindungen zwischen den Stärkepartikeln Schwachstellen sind, dass eine erfindungsgemäße Weichkapsel sogar bessere mechanische Eigenschaften aufweist, beispielsweise einen höheren E-Modul, als eine Weichkapsel der gleichen Zusammensetzung, welcher durch Plastifizierung der Stärke im Extruder hergestellt worden ist. Der Grund hierfür liegt zumindest teilweise darin, dass bei der Plastifizierung von Stärke infolge der hohen Temperatur und/oder der hohen Scherungen, das Molekulargewicht der Stärke Makromoleküle reduziert sind und die mechanischen Eigenschaften mit dem Molekulargewicht zunehmen. Da für die Herstellung des erfindungsgemäßen Films keine Scherung benötigt wird und deutlich tiefere Temperaturen als bei der Plastifizierung notwendig sind, entspricht das Molekulargewicht der Stärke in der Weichkapsel in etwa (Molekulargewichtsbestimmungen weisen meist einen beachtlichen Fehler auf, da die Messungen schwierig sind) dem Molekulargewicht der Stärke vor der Verarbeitung.

Bekannte Weichkapseln aus transparenter plastifizierter Stärke werden beim Lagern in Wasser weich und weiss (intransparent), bleiben aber mehr oder weniger formstabil und zerfallen bei geringer mechanischer Beanspruchung in Bruchstücke. Es findet keine Auflösung in ursprüngliche Stärkepartikel statt, da deren Identität bei der Plastifizierung mittels Extrusion zerstört worden ist.

Der hier ausgeführte Unterschied zwischen extrudierter Stärke und den erfindungsgemäßen Stärkeweichkapseln hat ausserdem den Vorteil, dass erfindungsgemäße Weichkapseln in Wasser gut zerfallen, makroskopisch gesehen sich auflösen (in die ursprünglichen Partikel), während die extrudierten Weichkapseln zwar weich werden, aber ihre Form ohne mechanische Einwirkung beibehalten. Somit erfolgt die Freisetzung des Kapselinhalts bei erfindungsgemäßen Kapseln leichter und es ist Kompatibilität mit Pharmakopöe Vorschriften gegeben, die eine Auflösung der Kapselhülle fordern.

Da die Partikel der Weichkapselhülle dicht gepackt sind, weist sie eine hohe Dichte auf. Sie liegt vorzugsweise im Bereich 1,07 - 1,3 g/cm³.

Bei Verwendung von Gießmassen, welche keine die Transparenz vermindernde Zusatzstoffe wie beispielsweise Pigmente aufweisen, wird die Gießmasse, welche Stärkepartikeln aufweist und darum praktisch vollständig intransparent ist, in dem Masse, wie die Verfestigung fortschreitet, zunehmend transparent. Nach Abschluss der Verfestigung ist die Weichkapsel dann praktisch vollständig transparent. Dies bedeutet, dass eine Schrift, welche von einer Person in einem Abstand gerade noch gelesen werden kann, von dieser Person in demselben Abstand immer noch gelesen werden kann, wenn sie mit einem transparenten Film (ca. 0,5 mm Dicke) zur Herstellung der Weichkapsel abgedeckt wird, und die Schriftgrösse höchstens um 50% vergrössert wurde.

Erfindungsgemäße Weichkapseln sind ein einem breiten Bereich von Luftfeuchtigkeiten und Temperaturen stabil, während Gelatineweichkapseln bei hohen Luftfeuchtigkeiten sehr weich werden und bei hohen Temperaturen schmelzen. Sie weisen eine geringere Sauerstoffpermeabilität auf als Gelatineweichkapseln.

Die guten mechanischen Eigenschaften der erfindungsgemäßen Weichkapseln sind eine Folge der Struktur des Weichkapsel Films als Agglomerat von dicht gepackten, destrukturierten Stärkekörnern, sowie eine Folge des hohen Molekulargewichts der Stärke, das durch das erfindungsgemäße Verfahren ermöglicht wird. Die destrukturierten Stärkekörner weisen eine gewisse Festigkeit auf und leisten daher einen Beitrag zu den guten mechanischen Eigenschaften der Weichkapsel in einem breiten Bereich von Luftfeuchtigkeiten.

### Verkapselungsvorrichtung

Das beschriebene Verfahren eignet sich gut zur Herstellung von Weichkapseln, da das Verfahren dem Gießen von Gelatine sehr ähnlich ist. Wenn von Gelatine auf Stärke umgestellt wird, kann dies durch einen Umbau mit vertretbaren Mitteln geschehen und der Umbau betrifft im wesentlichen nur den Teil der Verkapselungsvorrichtung, der die Herstellung des Films betrifft. Für die weiteren Verfahrensschritte müssen zwar gewisse Verfahrensparameter angepasst werden, aber es ergeben sich keine grundsätzlichen Änderungen. Vorteilhaft ist darüber hinaus, dass der Arbeitsschritt der Herstellung der Gelatineschmelze entfällt bzw. durch den deutlich einfacheren und raschen Arbeitsschritt der Herstellung der Stärkemischung ersetzt werden kann. Diese Gießmischung wird sehr einfach erhalten, indem die Komponenten unter Rühren miteinander gemischt werden, wobei übliche, einfache Rührwerke ausreichend sind. Für den Gießvorgang kann dieselbe Spreader Box verwendet werden, die beim Gießen von Gelatine eingesetzt wird.

Eine erfindungsgemäße Vorrichtung zur Herstellung von Weichkapseln auf Stärkebasis, weist daher folgende Vorrichtungen auf: eine Formgebungsvorrichtung, um eine Formgebung einer Stärkemasse zu einem Film zu ermöglichen, mindestens eine Heizvorrichtung, um bei und/oder nach der Formgebung eine Wärmebehandlung zur Gelierung der Stärke durchzuführen, sowie nach der Heizvorrichtung eine Rotary Die Vorrichtung, womit die Kapseln geformt, gefüllt und entformt werden. Gegebenenfalls weist die erfindungsgemäße Vorrichtung zur Herstellung von Weichkapseln noch eine Vorrichtung auf, um den Wassergehalt des Films bei und/oder nach der Formgebung zu regeln, insbesondere während der Verfestigung der Stärke im Bereich der Heizvorrichtung.

Der charakteristische Unterschied zwischen der Herstellung von Gelatine- und Stärkeweichkapseln besteht darin, dass die geschmolzene Gelatinegießmasse sich nach dem Gießen durch Abkühlung verfestigt, bzw. geliert, während die Stärkegießmasse sich nach dem Gießen umgekehrt durch Temperaturerhöhung verfestigt. Beim Gelatineverfahren wird die Masse bei rund 80°C auf eine zylinderförmige, gekühlte Trommel gegossen (typische Temperatur ca. 18°C), während beim erfindungsgemäßen Verfahren vorzugsweise auf ein rotierendes Verfahrensteil, gegossen wird, wobei dort die Temperaturerhöhung der Gießmasse erfolgt, insbesondere durch Wärmeleitung. Grundsätzlich kann jedoch alternativ oder zusätzlich jede andere Art von Heizen eingesetzt werden, wobei insbesondere Heizmethoden mittels Strahlung geeignet sind, beispielsweise Infrarotstrahlung oder Mikrowellenstrahlung. Weitere Heizmethoden verwenden Wasserdampf. Bevorzugt ist das rotierende Verfahrensteil eine Trommel.

Der Film bleibt bevorzugt bis zur im wesentlichen vollständigen Verfestigung (primäre Verfestigung) des Films in Kontakt mit dem rotierenden Verfahrensteil.

In einer bevorzugten Ausführung bleibt der Film auf mindestens 30% des Umfangs des rotierenden Verfahrensteils, besonders bevorzugt auf mindestens 40%, noch bevorzugter auf mindestens 50%, noch bevorzugter auf mindestens 60% ganz besonders bevorzugt auf mindestens 70%, in Kontakt mit dem rotierenden Verfahrensteil.

In einer bevorzugten Ausführung regelt die Vorrichtung zur Regelung des Wassergehalts des Films den Wassergehalt so, dass der Wassergehalt des Films während dem Kontakt mit dem rotierenden Verfahrensteil höchstens um 25 Gew.-%, besonders bevorzugt um höchstens 20 Gew.-%, noch bevorzugter um höchstens 15 Gew.-%, noch bevorzugter um höchstens 10 Gew.-%, noch bevorzugter um höchstens 7 Gew.-%, noch bevorzugter um höchstens 5 Gew.-%, ganz besonders bevorzugt um höchstens 3 Gew.-% reduziert (zur Verdeutlichung: liegt der Wassergehalt nach der Umformung der Gießmasse zu einem Film bei 40%, so liegt der nach einer Reduktion von 3% bei 37%).

In einer bevorzugten Ausführung ist das rotierende Verfahrensteil auf eine Temperatur von mindestens 25°C heizbar, besonders bevorzugt von mindestens 50°C, noch bevorzugter von mindestens 80°C, noch bevorzugter von mindestens 90°C, noch bevorzugter von mindestens 100°C, am bevorzugtesten von mindestens 105°C.

Bevorzugt weist das rotierende Verfahrensteil mindestens auf einer Seite eine Wärmeisolation auf.

Die Vorrichtung zur Regelung des Wassergehalts des Films nach der Formgebung weist in einer bevorzugten Ausführungsform ein Mittel auf, das den Film auf dem rotierenden Verfahrensteil auf mindestens 30% des Umfangs abgedeckt, besonders bevorzugt auf mindestens 40%, noch bevorzugter auf mindestens 50%, noch bevorzugter auf mindestens 60%, ganz besonders bevorzugt auf mindestens 70%. Damit wird der Wassergehalt im Film während der Verfestigung geregelt, insbesondere im wesentlichen konstant gehalten.

Bevorzugt wird diese Abdeckung durch ein mit-umlaufendes Band erreicht, das auf dem Film aufliegt und insbesondere dieselbe Geschwindigkeit oder Winkelgeschwindigkeit wie das rotierende Verfahrensteil aufweist. Dieses Band kann einen eigenen Antrieb aufweisen, bevorzugt wird es direkt mit dem rotierenden Verfahrensteil angetrieben, wobei die Kraftübertragung zwischen dem rotierenden Verfahrensteil bzw. dem Film und dem Band mittels Haftung erfolgt. Das Band kann, bevor es auf das rotierende Verfahrensteil bzw. den Film zu liegen kommt, beheizt werden, beispielsweise durch Strahlung wie Infrarotstrahlung.

Entlang dem Umfang des Bandes um das rotierende Verfahrensteil, in dem Bereich wo das Band aufliegt, können eine weitere oder mehrere Heizeinrichtungen zum Einsatz kommen, beispielsweise Infrarotstrahler.

Der verfestigte Film wird anschliessend gegebenenfalls gekühlt und dann analog einem Gelatinefilm weiterverwendet, beispielsweise geölt und dann mittels Rotary Dies für die Verkapselung eingesetzt.

Die Vorrichtung zur Regelung des Wassergehalts des Films nach der Formgebung weist in einer weiteren bevorzugten Ausführungsform ein Mittel auf, um den Raum über dem Film entlang mindestens eines Teils des rotierenden Verfahrensteils einzuschränken, sodass das Volumen, wohinein Wasser aus dem Film abdampft, beschränkt ist. Diese Einschränkung betrifft vorzugsweise mindestens 30% des Umfangs des rotierenden Verfahrensteils, besonders bevorzugt mindestens 40%, noch bevorzugter mindestens 50%, noch bevorzugter mindestens 60%, ganz besonders bevorzugt mindestens 70%. Vorzugsweise beträgt das eingeschränkte Volumen höchstens das 10-fache des Volumens des Films innerhalb der Einschränkung, besonders bevorzugt höchstens das 5-fache, noch bevorzugter höchstens das 2-fache. In einer bevorzugten Ausführungsform wird das eingeschränkte Volumen klimatisiert, d.h. Luftfeuchtigkeit und gegebenenfalls Temperatur werden geregelt.

Die Vorrichtung zur Regelung des Wassergehalts des Films nach der Formgebung weist in einer weiteren bevorzugten Ausführungsform ein Mittel auf, um dem Film Wasser zuzuführen, bevorzugt heisses Wasser, besonders bevorzugt Wasserdampf.

Die Vorrichtung zur Regelung des Wassergehalts des Films nach der Formgebung weist in einer weiteren bevorzugten Ausführungsform ein Mittel auf, um die Oberfläche des Films mit einer Flüssigkeit abzudecken. Insbesondere erzeugt das Mittel einen Film der Flüssigkeit auf dem Stärke Film oder das Mittel weist ein Bad der Flüssigkeit auf, wohindurch der Stärke Film geführt wird. Bevorzugt ist die Flüssigkeit ein Öl.

### Kurze Beschreibung der Zeichnungen

Figur 1 zeigt einen Ausschnitt einer ersten Ausführungsform der erfindungsgemäßen Vorrichtung zur Herstellung von Weichkapseln.
Figur 2 zeigt einen Ausschnitt einer zweiten Ausführungsform der erfindungsgemäßen Vorrichtung zur Herstellung von Weichkapseln.
Figur 3 zeigt eine erfindungsgemäße Vorrichtung zur Herstellung von Weichkapseln.
Figur 4 zeigt eine lichtmikroskopische Aufnahme eines erfindungsgemäßen Stärkefilms zur Herstellung einer Weichkapsel gemäß Beispiel 1, welcher bei einer relativen Luftfeuchtigkeit von 58 % gelagert wurde in der Vergrößerung 1:150 (es wir ein Filmausschnitt mit einer Breite von 0,57 mm gezeigt).
Figur 5 zeigt eine lichtmikroskopische Aufnahme eines erfindungsgemäßen Stärkefilms zur Herstellung einer Weichkapsel gemäß Beispiel 1 mit dem Vergrößerungsfaktor 150 (es wird ein Filmausschnitt mit einer Breite von 0,57 mm gezeigt), welcher bei einer relativen Luftfeuchtigkeit von 58 % gelagert wurde.
Figur 6 zeigt eine lichtmikroskopische Aufnahme eines nicht erfindungsgemäßen extrudierten, Stärkefilms zur Herstellung einer Weichkapsel gemäß dem Patent EP 1 103 254 B1 mit dem Vergrößerungsfaktor 150 (es wird ein Filmausschnitt mit einer Breite von 0,57 mm gezeigt).
Figur 7 zeigt eine lichtmikroskopische Aufnahme einer wässerigen Suspension von unverarbeiteter, doppelbrechender, hydroxypropylierter Tapiocastärke mit dem Vergrößerungsfaktor 150 (es wird ein Ausschnitt mit einer Breite von 0,57 mm gezeigt).
Figur 8 zeigt eine lichtmikroskopische Aufnahme einer wässerigen Suspension von hydroxypropylierter Tapiocastärke, welche auf 70°C erhitzt wurde, mit dem Vergrößerungsfaktor 150 (es wird ein Ausschnitt mit einer Breite von 0,57 mm gezeigt).
Figur 9 zeigt eine lichtmikroskopische Aufnahme einer wässerigen Suspension von hydroxypropylierter Tapiocastärke, welche erhalten wurde, indem eine erfindungsgemäße Weichkapsel gemäß Beispiel 1 in Wasser auf 70°C erhitzt wurde, mit dem Vergrößerungsfaktor 150 (es wird ein Ausschnitt mit einer Breite von 0,57 mm gezeigt).
Figur 10 zeigt eine lichtmikroskopische Aufnahme einer wässerigen Suspension von nichtverarbeiteter hydroxypropylierter Kartoffelstärke unter gekreuzten Polarisatoren, mit dem Vergrößerungsfaktor 150 (es wird ein Ausschnitt mit einer Breite von 0,57 mm gezeigt).
Figur 11 zeigt eine lichtmikroskopische Aufnahme einer wässerigen Suspension von hydroxypropylierter Kartoffelstärke, welche auf 70°C erhitzt wurde, mit dem Vergrößerungsfaktor 150 (es wird ein Ausschnitt mit einer Breite von 0,57 mm gezeigt).
Figur 12 zeigt eine lichtmikroskopische Aufnahme einer wässerigen Suspension von hydroxypropylierter Kartoffelstärke, welche erhalten wurde, indem eine Probe einer erfindungsgemäßen Weichkapsel gemäß Beispiel 5 in Wasser auf 70°C erhitzt wurde, mit dem Vergrößerungsfaktor 150 (es wird ein Ausschnitt mit einer Breite von 0,57 mm gezeigt).
Figur 13 zeigt die Molmassenverteilungen einer Ausgangsstärke und einer Stärke, welche durch Auflösen einer erfindungsgemäßen Weichkapsel gemäß Beispiel 5 zurück gewonnen wurde, welche aus dieser Ausgangsstärke hergestellt wurde.

### Beispiele

Die Rezepturen zu den Beispielen sind in der Tabelle 1 aufgeführt. Es wurden jeweils Gießmischungen von 10 kg hergestellt. Die Viskosität der Gießmischung, die mechanischen Eigenschaften und die Wiedergewinnung der Stärke sind ebenfalls in der Tabelle 1 aufgeführt.

Bei allen Versuchen zur Herstellung von Weichkapseln wurden vollständig transparente Weichkapseln von guter Qualität erhalten, insbesondere waren sie formstabil, wiesen sehr gute Schweissnähte auf, waren einfach zu reinigen und zu trocknen.

Bei allen erfindungsgemäßen Beispielen zeigte die mikroskopische Analyse, dass die Stärkefilme aus dicht gepackten, destrukturierten Stärkekörnern (< 5% doppelbrechende Stärkekörner) aufgebaut waren und die Filme konnten in Wasser wieder in diese Bestandteile aufgelöst werden, d.h. nach Zerfall der Filme konnten im Wasser die destrukturierten Stärkekörner wieder nachgewiesen und deren Gewicht bestimmt werden (Wiedergewinnungsverfahren Nr. 1).

Der Zerfall der Weichkapseln wurde im Schüttelbad bei 37°C in 0,5%iger Salzsäure an Weichkapseln bestimmt, welche nach der Herstellung auf rund 10% Wassergehalt getrocknet worden sind und anschliessen 20 Tage bei 33% Luftfeuchtigkeit gelagert wurden. Bei allen Beispielen erfolgte die Freisetzung des Inhalts der Weichkapseln nach weniger als 20 min.

### Beispiel 1

Gemäß der Rezeptur 1 wurde zunächst in einen heizbaren und evakuierbaren Kessel mit Rührwerk bei Raumtemperatur das Wasser und der Weichmacher zugegeben und diese beiden Komponenten bei 100rpm gemischt. Dann wurde die bei einem Wassergehalt von 35% sehr schonend extrudierte Stärke S1E zugeführt und während 5min bei 100rpm in der Mischung von Wasser und Weichmacher gelöst. Die extrudierte Stärke S1E wurde mittels einer Schlagmühle aus getrocknetem Extrudat (basierend auf der Stärke S1) hergestellt und wies eine Partikelgrössenverteilung im Bereich von 30 - 150 µmm, sowie einen Anteil von 10% an kurzkettiger Amylose auf (diese kurzkettige Amylose wurde durch vollständige Entzweigung mittels Pullulanase von Tapiocastärke erhalten und wies ein Zahlenmittel des Polymerisationsgrades DPn von 25 auf).

Zu dieser Mischung wurde dann die granuläre Stärke S1, welche ein Gewichtsmittel des Molekulargewichts M_{w} von 30.100.000g/mol aufwies, zugeführt und während 5min bei 100rpm darin dispergiert, wonach diese Mischung auf 45°C erwärmt und 5min bei 100rpm durch Anlegen von Vakuum entgast wurde (Entfernen von Luftblasen). Die dynamische Viskosität dieser Mischung lag bei dieser Temperatur bei 5,7 Pas bei einer Scherrate von 1,1/s.

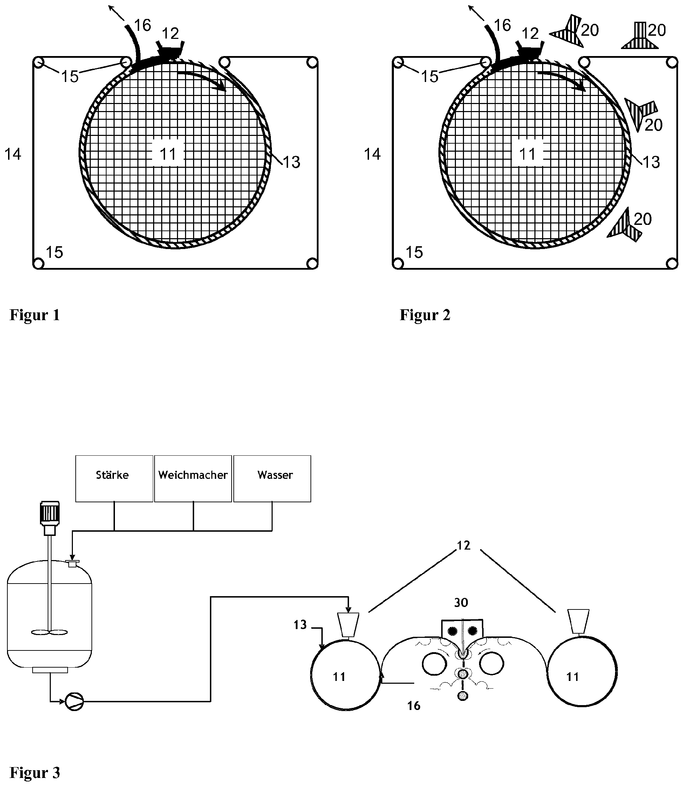

Die warme Mischung wurde dann mittels einer erfindungsgemäßen Gießvorrichtung zu einem Film verarbeitet. Die Vorrichtung ist in Figur 1 dargestellt. Sie umfasst einer rotierende, beheizte Trommel (11), eine Spreader Box (12), ein umlaufendes Teflonband (14), und Umlenkrollen (15). Die Gießmasse (13) wird zu einem Film (16) verfestigt.

Die Trommel (11) besteht aus einem Metallzylinder von 50 cm Durchmesser, der mittels einer Heizflüssigkeit auf die Temperatur TZ von 105°C geheizt wurde. Die Drehzahl n der Trommel lag bei 0,6 Umdrehungen pro Minute. Die Gießtemperatur TG der Mischung lag bei 45°C. Die Mischung wurde mittels der Spreader Box (12) auf den rotierenden Metallzylinder zu einem Film (16) mit 25 cm Breite und 0,7 mm Dicke gegossen. Entlang ¾ des Umfangs wurde der gegossene Film (16) mit dem mitrotierenden Teflonband (14) abgedeckt, damit der Wassergehalt im Film konstant blieb. Nach einer ¾ Umdrehung wurde der Film vom Metallzylinder abgelöst, und in eine Rotary Die Vorrichtung (30) von CS-J1-500R von Chang Sung weitertransportiert, in welcher er bei 2 rpm der Rotary Dies zu Weichkapseln der Form/Größe Oval#10, gefüllt mit Sojaöl, weiterverarbeitet wurde (siehe Figur 3). Die erhaltenen Weichkapseln waren vollständig transparent und die Weichkapselhälften waren gut miteinander verschweisst, die frischen Kapseln zeigten gute Formstabilität und liessen sich im Tumbler reinigen und trocknen. Es wurden keine doppelbrechenden Stärkekörner in den Weichkapseln beobachtet. Die Massentemperatur des Films auf der Trommel lag nach ¾ Umdrehung bei 91°C.

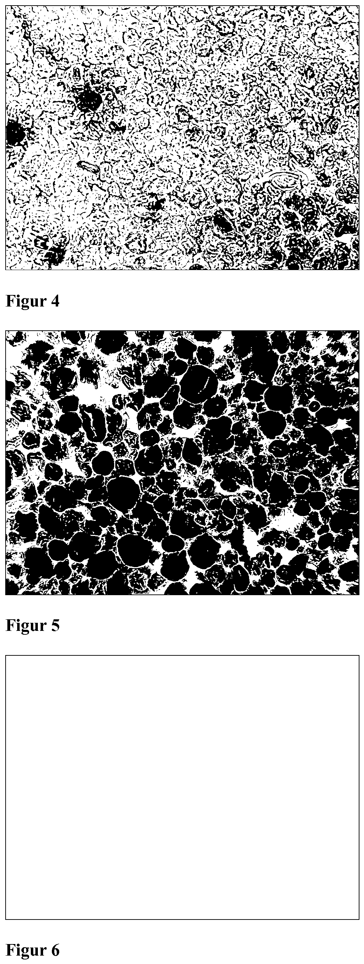

Eine lichtmikroskopische Aufnahme eines Stärkefilms, welcher 7 Monate über Natriumbromid (relative Luftfeuchtigkeit von 58 %) gelagert wurde ist in Figur 4 gezeigt. Es ist gut zu erkennen, dass der Film aus miteinander verbundenen Stärkekörnern besteht. Figur 5 zeigt eine lichtmikroskopische Aufnahme eines Stärkefilms, welcher 7 Monate über Magnesiumchlorid (relative Luftfeuchtigkeit von 33 %) gelagert wurde. Zum Vergleich wird in Figur 6 ein extrudierter Stärkefilm gemäß dem Patent EP 1 103 254 B1 gezeigt. Durch die Extrusion wurden alle Stärkepartikel zerstört, so dass sie lichtmikroskopisch nicht mehr nachweisbar sind.

Die E-Moduli von Filmen aus Beispiel 1, welche 2 Wochen bei relativen Luftfeuchtigkeiten von 33%, 43%, 57% und 75% gelagert worden sind, lagen bei 23MPa, 3,4MPa, 3.7MPa und 3,3MPa, während die E-Moduli von Filmen derselben Zusammensetzung, welche jedoch mittels Extrusion hergestellt worden sind, in Längsrichtung bei denselben Luftfeuchtigkeiten bei 4.5 MPa, 0,7 MPa, 0,9 MPa und 0.4MPa lagen.

### Beispiel 1a

Beispiel 1 wurde wiederholt. Die extrudierte Stärke S1E und die granuläre Stärke wurden zusammen zur Mischung von Wasser und Weichmacher zugemischt. Es zeigte sich, dass die Reihenfolge bei der Herstellung der Gießmischung ohne Effekt auf die weitere Verarbeitung und die Produkteigenschaften bleibt.

### Beispiel 1b

Beispiel 1 wurde wiederholt. Die fertige Gießmischung wurde bei Raumtemperatur zwei Stunden vor der Weiterverarbeitung gelagert, ohne dass dies einen Effekt auf die weitere Verarbeitung oder die Produkteigenschaft hatte.

### Beispiel 1c

Beispiel 1 wurde wiederholt. Die fertige Gießmischung wurde bei 45°C zwei Stunden vor der Weiterverarbeitung gelagert, ohne dass dies einen Effekt auf die weitere Verarbeitung oder die Produkteigenschaft hatte.

### Beispiel 2

Analog Beispiel 1, statt 38% Wasser wies die Gießmasse einen Wassergehalt von 35% auf. Die Temperatur der Trommel wurde auf 108°C eingestellt. Die Massentemperatur des Films auf der Trommel lag nach ¾ Umdrehung bei 93°C.

### Beispiel 3

Analog Beispiel 1, statt 38% Wasser wies die Gießmasse einen Wassergehalt von 41,1% auf. Die Temperatur der Trommel wurde auf 103°C eingestellt. Die Massentemperatur des Films auf der Trommel lag bei ¾ Umdrehung bei 89°C.

### Beispiel 4

Analog Beispiel 1, der Anteil der extrudierten Stärke S1E in der Gießmasse wurde von 2,28% auf 4,49% erhöht, wodurch die dynamische Viskosität bei 45°C bei einer Scherrate von 1,1/s von 5,7Pas auf 21Pas anstieg. Die Temperatur der Trommel wurde auf 105°C eingestellt. Die Massentemperatur des Films auf der Trommel lag bei ¾ Umdrehung bei rund 90°C.

### Beispiel 5

Analog Beispiel 1, die hydroxypropylierte, vernetzte Tapiocastärke S1 wurde durch die native Tapiocastärke S2 und die Stärke S1E durch die pregelatinisierte Stärke S2P ersetzt. Die Temperatur der Trommel wurde auf 111°C eingestellt. Die Massentemperatur des Films auf der Trommel lag nach ¾ Umdrehung bei rund 96°C.

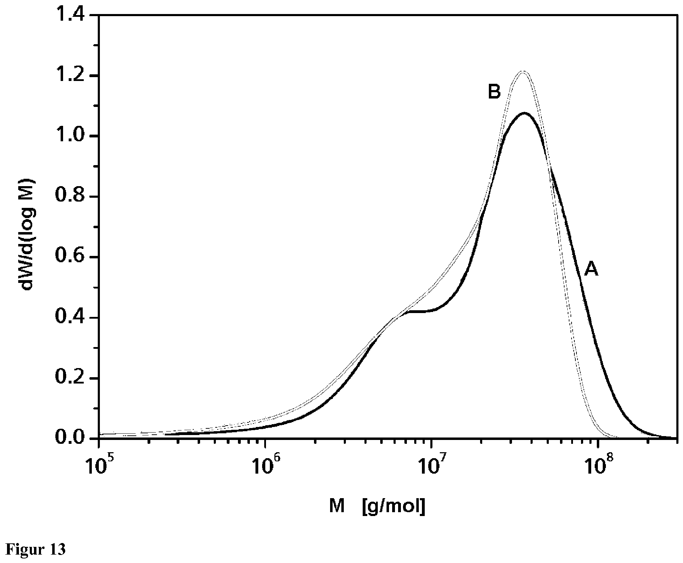

Die Stärken S2 und S2P wiesen vor der Verarbeitung ein Gewichtsmittel des Molekulargewichts M_{w} von 22.690.000g/mol auf und die aus den damit hergestellten Weichkapseln extrahierte Stärke wies ein Molekulargewicht M_{w} von 21.340.000 auf, d.h. das Molekulargewicht wurde bei der Herstellung der Weichkapseln nur minimal reduziert (vergl. Figur 13).

### Beispiel 6

Analog Beispiel 1, der Glyceringehalt wurde erhöht. Die Temperatur der Trommel wurde auf 102°C eingestellt. Die Temperatur des Films nach ¾ Umdrehungen lag bei 88°C.

### Beispiel 7

Analog Beispiel 1, die hydroxypropylierte, vernetzte Tapiocastärke S1 wurde durch die native Waxy Kartoffelstärke S4 ersetzt. Die Temperatur der Gießmischung lag bei 40°C. Die Temperatur der Trommel wurde auf 102°C eingestellt. Die Temperatur des Films nach ¾ Umdrehungen lag bei 87°C.

### Beispiel 8

Analog Beispiel 1, die hydroxypropylierte, vernetzte Tapiocastärke S1 wurde durch die hydroxypropylierte Kartoffelstärke S5 und die Stärke S1E durch die pregelatinisierte hydroxypropylierte Kartoffelstärke S5P ersetzt. Die Temperatur der Gießmischung lag bei 40°C. Die Temperatur der Trommel wurde auf 101°C eingestellt. Die Temperatur des Films nach ¾ Umdrehungen lag bei 86°C.

Die Stärken S5 und S5P wiesen vor der Verarbeitung ein Gewichtsmittel des Molekulargewichts M_{w} von 13.530.000g/mol auf und die aus den damit hergestellten Weichkapseln extrahierte Stärke wies bei einer ersten Messung ein Molekulargewicht M_{w} von 13.490.000, bei einer zweiten Messung von 15.460.000 auf, d.h. das Molekulargewicht wurde bei der Herstellung der Weichkapseln praktisch nicht verändert. Die anscheinende Erhöhung des Molekulargewichts bei der zweiten Messung dürfte darauf zurückzuführen sein, dass die Genauigkeit von Molekulargewichtsmessungen bei den hohen Molekulargewichten begrenzt ist.

### Beispiel 9

Analog Beispiel 1, die extrudierte Stärke S1E wurde durch die pregelatinisierte Stärke S1P ersetzt.

Die Stärken S1 und S1P weisen ein Gewichtsmittel des Molekulargewichts M_{w} von 30.100.000g/mol auf. Die Molekulargewichtsanalyse für die Stärke in den entsprechenden Weichkapseln ergab in einer ersten Messung ein Molekulargewicht M_{w} von 21.340.000g/mol, und in einer zweiten Messung ein Molekulargewicht M_{w} von 20.220.000g/mol, d.h. das Molekulargewicht wurde durch das Verfahren nur geringfügig reduziert. Insbesondere im Vergleich zum Extrusionsverfahren, wo die Stärke S1, obwohl unter möglichst schonenden Bedingungen extrudiert, d.h. bei hohem Wassergehalt und tiefen Schergeschwindigkeiten, nur noch ein Molekulargewicht M_{w} von 920.000g/mol aufwies.

### Beispiel 9a

Beispiel 9 wurde wiederholt. Die extrudierte Stärke S1E wurde durch die Stärke S1 (als gelöste Stärke) ersetzt und nach Zugabe dieser Stärke S1 zum Gemisch aus Wasser und Weichmacher wurde diese Stärke S1 in diese Mischung durch Erhitzen auf 90°C destrukturiert. Nach anschliessendem Abkühlen auf eine Temperatur unterhalb 45°C wurde dann die granuläre Stärke S1 (als granuläre Stärke) zugemischt. Dies hatte keinen Effekt auf das nachfolgende Verfahren und die Produkteigenschaften.

### Beispiel 9b

Beispiel 9a wurde wiederholt. Um das Abkühlen zu vermeiden, wurde das Verfahren vereinfacht, indem die Stärke S1 (als gelöste Stärke) nur in einem Teil des Wasser-Weichmacher Gemisches destrukturiert wurde und nachfolgend der Rest von Wasser und Weichmacher bei Raumtemperatur zugeführt wurde, um die Temperatur auf unterhalb 45°C zu senken.

### Beispiel 10

Analog Beispiel 9. Die pregelatinisierte Stärke S1P wurde durch die pregelatinisierte Stärke S6P ersetzt. Auch hier sind dieselben Verfahrensweisen wie in den Beispielen 9a und 9b beschrieben anwendbar, um die Stärke S6 (als gelöste Stärke) zu destrukturieren.

### Beispiele 11 bis 13

Analog Beispiel 1. Bei diesen Beispielen wurde die gelöste Stärke S1E durch Verdickungsmittel V1, V2 und V3 ersetzt, wodurch das Zerfallsverhalten der Stärke Weichkapseln im sauren wässrigen Milieu beschleunigt werden konnte. Um die Verdickungsmittel V2 (Xanthan) und V3 (Locust Bean Gum, Johannisbrotkern Mehl) in der Mischung von Wasser und Weichmacher zu lösen, wurde analog Beispiel 9a die Mischung von Wasser, Weichmacher und Polysaccharid auf 90°C erhitzt und dann auf eine Temperatur unterhalb rund 45°C abgekühlt, bevor die granuläre Stärke zugemischt wurde. Auch hier ist dieselbe Variante wie in Beispiel 9b anwendbar, um ein aktives Kühlen der Mischung aus Wasser, Weichmacher und gelöstem Polysaccharid zu vermeiden.

### Beispiele 14 bis 16

Analog Beispiel 1. Bei diesen Beispielen wurde die gelöste Stärke S1E durch verschiedene Tapiocadextrine S7, S8 und S9 ersetzt, wodurch das Zerfallsverhalten der Stärkeweichkapseln im sauren wässrigen Milieu beschleunigt werden konnte. Um die Dextrine S7 und S8 in der Mischung von Wasser und Weichmacher zu lösen, wurde analog Beispiel 9a die Mischung von Wasser, Weichmacher und Polysaccharid auf 90°C erhitzt und dann auf eine Temperatur unterhalb rund 45°C abgekühlt, bevor die granuläre Stärke (Stärke 1 gemäß Tabelle 1) zugemischt wurde. Auch hier ist dieselbe Variante wie in Beispiel 9b anwendbar, um ein aktives Kühlen der Mischung aus Wasser, Weichmacher und Stärke zu vermeiden.

### Beispiel 17

Bei allen aus den Beispielen 1 bis 17 erhaltenen Stärkeweichkapseln, liessen sich die ursprünglichen Stärkepartikel durch Einlegen in Wasser zurück gewinnen und durch Anfärben mit Lugolscher Lösung unter dem Mikroskop sichtbar machen.

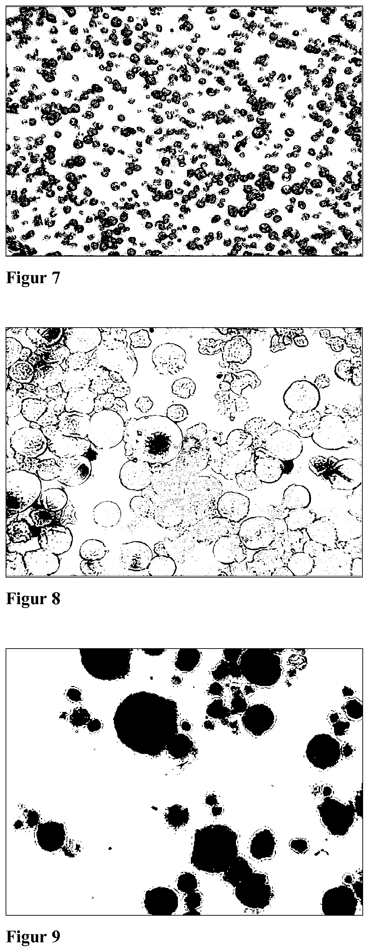

Eine lichtmikroskopische Aufnahme der unverarbeiteten, granulären Tapiocastärke S1 aus Beispiel 1 findet sich in Figur 7.

Figur 8 zeigt die Veränderung dieser Stärke unter Temperatureinfluss. Die Probe wurde hergestellt, indem 20 Gew.-% Stärke in Wasser in einem Reagenzglas suspendiert und 5 min im Wasserbad auf 70°C aufgeheizt wurden. Nach dem Abkühlen auf Raumtemperatur wurde die Stärke mit Jod angefärbt und mikroskopiert.. Während Figur 7 kleine doppelbrechende Stärkepartikel zeigt, erkennt man, dass die Partikel in Figur 8 gequollen sind und keine Doppelbrechung mehr zeigen.

Figur 9 zeigt Stärkepartikel, welche aus Weichkapseln zurück gewonnen wurden. Hierzu wurden Weichkapseln aus Beispiel 1 zunächst 7 Monate über Magnesiumchlorid (relative Luftfeuchtigkeit: 33 %) gelagert. Eine Probe wurde hergestellt, indem rund 100mg der Weichkapsel in 7g Wasser unter Rühren mit einem Magnetrührer 30 min bei 70°C gehalten wurde, wobei das Material in Partikel zerfiel. Nach Abkühlung wurde mit Iod gefärbt wurde. Diese Stärkepartikel aus dem Film sind stärker gefärbt und stärker verdünnt, unterscheiden sich aber nicht wesentlich von jenen in Figur 8, welche durch Erhitzen der suspendierten Stärke erhalten werden konnten. Damit wird gezeigt, dass die Weichkapsel aus destrukturierten Stärkekörnern besteht.

### Beispiel 18

Beispiel 17 wurde mit der Kartoffelstärke S5 und den Weichkapseln gemäß Beispiel 8 wiederholt.

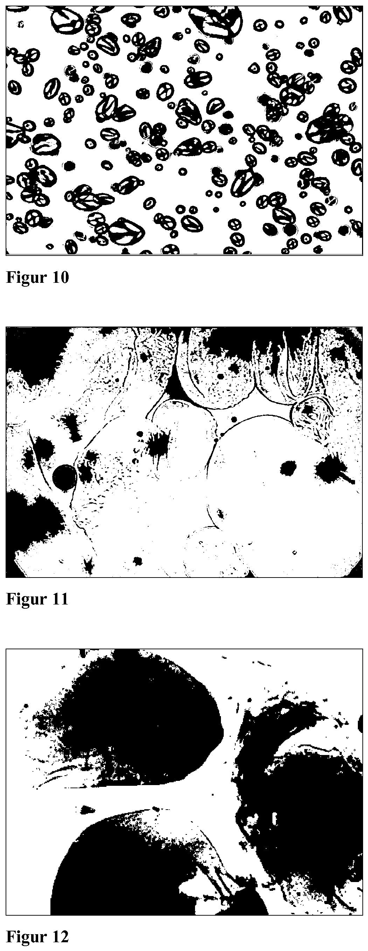

Eine lichtmikroskopische Aufnahme unter gekreuzten Polarisatoren der unverarbeiteten Stärke S5 aus Beispiel 8 findet sich in Figur 10. Bei den grösseren Körnern sind gut die bekannten Malteserkreuze zu erkennen, welche typisch sind für native Stärke.

Figur 11 zeigt die Veränderung dieser Stärke nach Erhitzen auf 70°C. Figur 12 zeigt Stärkepartikel, welche aus Weichkapseln gemäß Beispiel 8 zurück gewonnen wurden, welche 7 Monate über Natriumbromid (relative Luftfeuchtigkeit: 58 %) gelagert wurden.

Sie sind analog den Stärkekörnern von Figur 11, jedoch stärker gefärbt und stärker verdünnt. Hiermit wird gezeigt, dass die Weichkapsel aus destrukturierten Stärkekörnern besteht, die in eine Suspension überführt werden können und durch Sedimentation zurück gewonnen werden können.

### Beispiel 19

Figur 4 zeigt eine lichtmikroskopische Aufnahme eines erfindungsgemäßen Stärkefilms zur Herstellung einer Weichkapsel gemäß Beispiel 1. Vom Stärkefilm wurde mit der Rasierklinge eine sehr dünne Schicht abgetrennt und darauf ein Tropfen von Iod Lösung gegeben (die dunklen Stellen wurden stärker gefärbt). Dieses Präparat wurde dann zwischen zwei Objektträgern von Hand gepresst, um die Dicke des Films noch etwas zu reduzieren. Die resultierende Filmdicke wies etwa die Dicke von zwei Stärkekörnern auf, sodass die Körner teilweise übereinander liegen. Dennoch wird gut erkennbar, dass der Film aus einer dickten Packung von destrukturierten Stärkekörnern besteht (es war keine Doppelbrechung mehr erkennbar)

Figur 5 zeigt eine lichtmikroskopische Aufnahme eines erfindungsgemäßen Stärkefilms zur Herstellung einer Weichkapsel gemäß Beispiel 1. Um die individuellen Stärkekörner gegenüber Figur 4 deutlicher zu machen, wurde der mit der Rasierklinge erhaltene Stärkefilm bei 70°C kurz gequollen, die Stärkekörner mit Iod angefärbt und der Film zwischen zwei Objektträgern von Hand gepresst, sodass die Filmdicke etwa der Dicke der Körner entsprach. Infolge der Quellung bei 70°C sind die Körner angequollen und daher etwas grösser als bei Figur 4.

### Beispiel 20

Figur 6 zeigt eine lichtmikroskopische Aufnahme eines nicht erfindungsgemäßen, extrudierten, Stärkefilms zur Herstellung einer Weichkapsel gemäß dem Patent EP 1 103 254 B1 mit dem Vergrößerungsfaktor 150 (es wird ein Filmausschnitt mit einer Breite von 0,57 mm gezeigt). Da die Stärke vollständig gelöst worden ist, sind keine Partikel von Stärke mehr vorhanden. Mit dem Wiedergewinnungsverfahren 2 ergab sich noch ein Masseanteil von rund 1.5% des trockenen Films, der aus der Lösung sedimentiert werden konnte und auf nicht-lösliche Additive zurückgeführt werden kann.

### Beispiel 21

Die Molmassenverteilungen der unverarbeiteten Stärke S2 und der zu einer erfindungsgemäßen Weichkapsel verarbeiteten Stärke S2 gemäß Beispiel 5, wurden miteinander verglichen. Hierzu wurde die Auflösung der Stärkeprobe bzw. der Weichkapselprobe durch Druckkochung unter definierten Bedingungen in einem Miniautoklav und die Untersuchung der Molmassenverteilung der molekular dispers gelösten Stärke mittels GPC-MALLS durchgeführt.

Dazu wurden die Stärkeproben mit einer Konzentration von 3 Gew.-% Trockensubstanz in Wasser suspendiert. Diese Suspension wurde in einem Miniautoklav unter Rühren aufgeheizt. Nach Erreichen von 150°C wurde die Temperatur für 20 Minuten gehalten. Anschließend wurden die Lösung auf 60°C abgekühlt, auf 0,3 Gew.-% verdünnt, mit einem 5 µm Membranfilter filtriert und auf der GPC-MALLS gemessen.

Die erhaltenen Molmassenverteilungen sind in Figur 13 dargestellt, wobei A die Probe der Ausgangsstärke S2 und B die Weichkapselprobe gemäß Beispiel 5 bezeichnet. Als mittlere Molmasse der Ausgangsstärke ergibt sich M_{w} = 22,69 × 10⁶ g/mol und als Molmasse der Stärke, welche aus der Weichkapsel zurück gewonnen wurde, ergibt sich M_{w} = 21,84 × 10⁶ g/mol. Es kann festgestellt werden, dass die relativ hohe Molmasse der Ausgangsprobe durch die Verarbeitung zu einer Weichkapsel nicht signifikant abgebaut wurde. Ausgangsstärke und verarbeitete Stärke liegen in einem vergleichbaren Molmassenbereich.

### Messmethoden

Dynamische Viskositäten wurden mit Hilfe eines Brookfield Viskosimeters des Typs LVDV-I+ bei einer Scherrate von 1.1/s (5 rpm, Spindle 25) und den angegebenen Temperaturen bestimmt.

Die mechanischen Eigenschaften (Bruchdehnung, E-Modul) wurden auf einem Instron 5542 Prüfsystem gemäß ISO 527 gemessen.

Wassergehalte wurden durch Trocknung über Phosphorpentoxid bei 80°C während 48 h gemessen.

Die GPC-MALLS wurde mittels eines Alliance 2695 Separationsmoduls der Firma Waters, DRIDetektor. 2414 der Firma Waters und MALLS-Detektor Dawn-HELEOS von Wyatt Technologie Inc., Santa Barbara, USA, mit einer Wellenlänge 1 = 658 nm und einer K5 Durchflusszelle durchgeführt. Säulen: SUPREMA-Gel Säulensatz, Exclusionsgrenzen S30000 mit 108-106, S1000 mit 2×106-5×104, S100 mit 105- 103. Eluent: DMSO mit 0,09 m NaNO₃, Temperatur: 70°C, Auswertung: Astra Software 5.3.0.18. Für alle Proben wurde ein Brechungsindexinkrement dn/dc von 0.068 angenommen.

Die Bestimmung des unlöslichen Bestandteils im Film wird wie folgt durchgeführt: Vorgängig wurden die getrockneten Weichkapseln bei 57% Luftfeuchtigkeit 2 Monate gelagert. Eine Probemenge von 100 - 150mg (Trockenmasse M0) in Form eines Filmstücks der Weichkapselhülle von 0,5mm Dicke wird bei 70°C zusammen mit 7 g demineralisiertem Wasser in einem Reagenzglas während 30 min unter langsamem Rühren mit einem Magnetrührer gequollen und/oder gelöst. Danach wird das Reagenzglas zentrifugiert, bis die ungelösten Bestandteile sedimentiert haben und der Überstand klar geworden ist. Der Überstand wird dann dekantiert. Dann werden 7 g demineralisiertes Wasser zugegeben und mit dem Sediment verrührt, sowie erneut zentrifugiert und dann dekantiert. Dieser Vorgang wird nochmals wiederholt, um sicherzustellen, dass sich keine löslichen Bestandteile mehr im Sediment befinden. Dieses Sediment besteht bei einem Film bestehend aus Stärke und Weichmacher, aus nicht-gelöster Stärke. Schliesslich wird das Sediment während 48h bei 80°C über Phosphorpentoxid getrocknet und davon die trockene Masse (M1) bestimmt. Der Anteil der Masse, die nach dem Lösevorgang zurück gewonnen werden kann ergibt sich somit in Gew.-% zu 100 × M1/M0. Der Anteil der Stärke, die nach dem Lösevorgang zurück gewonnen werden kann ergibt sich bei einem Stärke Film bestehend aus Stärke und Weichmacher, in Gew.-% zu 100 × M1/(M0 × (1-(WM/100)), wobei WM der Anteil in Gew.-% des Weichmachers der trockenen Mischung ist. In der Regel weist der Stärkefilm neben den Stärkepartikeln höchstens noch minimale Anteile an unlöslichen Bestandteilen auf wie z.B. Pigmente (typischerweise < 0,5%) oder Füllstoffe wie Titandioxid (typischerweise < 1,5%). Solche Bestandteile werden bei der Trockenmasse MO und der Masse M1 gegebenenfalls subtrahiert.

**Tabelle 1**

| | | | | **Rezeptur der Gießmischung** | | | | | | | | **Frischer Film** | | | |
|---|---|---|---|---|---|---|---|---|---|---|---|---|---|---|---|
| **Beispiel** | **granuläre Stärke** | **gelöste Stärke** | **Verd. Mittel** | **granuläre Stärke** | **gelöste Stärke** | **SCA** | **Verd. Mittel** | **H₂O** | **WM** | **Viskosität der Gießmischung** | | **E-Modul** | **Dehnung** | **H₂O** | **Wg**. |
| | | | | [%] | [%] | [%] | [%] | [%] | [%] | **[°C]** | **[Pas]** | **[MPa]** | [%] | [%] | [%] |
| | | | | | | | | | | | | | | | |
| **1** | S1 | SIE | - | 38,97 | 2,28 | 0,25 | - | 38,0 | 20,5 | 45 | 5,7 | 0,03 | 386 | 36,5 | 86,3 |
| **2** | S1 | SIE | - | 40,85 | 2,39 | 0,27 | - | 35,0 | 21,5 | 45 | 14 | 0,04 | 430 | 34,2 | 87,2 |
| **3** | S1 | SIE | - | 37,06 | 2,17 | 0,24 | - | 41,1 | 19,5 | 45 | 2 | 0,02 | 531 | 40.0 | 86,7 |
| **4** | S1 | SIE | - | 36,54 | 4,49 | 0,50 | - | 38,0 | 20,5 | 45 | 21 | 0,03 | 420 | 37.0 | 82,3 |
| **5** | S2 | S2P | - | 38,97 | 2,28 | 0,25 | - | 38,0 | 20,5 | 45 | 2,8 | 0,03 | 452 | 36,8 | 92,1 |
| **6** | S1 | SIE | - | 39,08 | 2,29 | 0,25 | - | 33.5 | 24.9 | 45 | 21 | 0,02 | 510 | 33,1 | 91,5 |
| **7** | S4 | SIE | - | 38,98 | 2,28 | 0,25 | - | 38,0 | 20,4 | 40 | 8 | 0,14 | 148 | 35,8 | 73,4 |
| **8** | S5 | S5P | - | 39,42 | 1,88 | 0,21 | - | 38,0 | 20,5 | 40 | 4,3 | 0,03 | 430 | 36.5 | 64,2 |
| **9** | S1 | S1P | - | 39,43 | 2,09 | - | - | 38,0 | 20,5 | 45 | 11 | 0,04 | 421 | 36.7 | 88,4 |
| **10** | S1 | S6P | - | 39,45 | 2,09 | - | - | 38,0 | 20,5 | 45 | 4 | 0,02 | 523 | 34,3 | 79,5 |
| **11** | S1 | - | V1 | 41,11 | - | - | 0,41 | 38,0 | 20,5 | 45 | 35 | 0,05 | 440 | 37,9 | 89,2 |
| **12** | S1 | - | V2 | 41,32 | - | - | 0,21 | 38,0 | 20,5 | 45 | 17 | 0,04 | 508 | 37,7 | 91,2 |
| **13** | S1 | - | V3 | 41,11 | - | - | 0,41 | 38,0 | 20,5 | 45 | 20 | 0,04 | 467 | 37,8 | 92,5 |
| **14** | S1 | S7 | - | 33,24 | 8,30 | - | - | 38,0 | 20,5 | 45 | 13 | 0,03 | 507 | 37,2 | 88,4 |
| **15** | S1 | S8 | - | 33,23 | 8,30 | - | - | 38,0 | 20,5 | 45 | 10 | 0,02 | 563 | 37,4 | 86,2 |
| **16** | S1 | S9 | - | 36,56 | 5,02 | - | - | 38,0 | 20,5 | 45 | 22 | 0,02 | 499 | 35 | 84,7 |

### Legende zu Tabelle 1

granuläre Stärke:
- S1: hydroxypropylierte, vernetzte Tapioca Stärke (Creamtex 75725 von Cerestar)
- S2: Native Tapioca Stärke (von Cerestar)
- S4: Waxy Kartoffel Stärke (Eliane 100 von AVEBE)
- S5: hydroxypropylierte Kartoffelstärke (Emden KH 15 von Emsland)
gelöste Stärke:
- S1E: Stärke S1, extrudiert, enthaltend 10% kurzkettige Amylose
- S1P: Stärke S1, pregelatinisiert
- S2P: Stärke S2, pregelatinisiert
- S5P: Stärke S5, pregelatinisiert
- S6P: hydroxypropylierte Stärke (Emcol H7 von Emsland), pregelatinisiert
- S7: Tapioca Dextrin (Cleargum TA 90 von Roquette)
- S8: Tapioca Dextrin (Tapioca Dextrin 11 von Tate&Lyle)
- S9: Mischung aus 50% Stärke S1P und 50% Tapioca Dextrin (Dextrin D-400 von Cerestar)

Verd. Mittel (Verdickungsmittel):
- V1: Guar-Gummi (Meypro Guar CSAA M-200 von Meyhall/Rhodia)
- V2: Xanthan-Gummi (Keltrol HP E415 von Kelko)
- V3: Johannisbrotkernmehl (Meypro LBG Fleur M-175 von Meyhall/Rhodia)

- WM:: Glycerin als Weichmacher

alle %-Angaben in Gew.-% bezogen auf 100 Gew.-% der gesamten Gießmischung

Die mechanischen Eigenschaften (E-Modul und Dehnung) des frischen Films wurden bei einer Temperatur von 25°C, 10 min nach der Herstellung des Films gemessen.
- Wg.:: Wiedergewinnung gemäß Wiedergewinnungsverfahren Nr. 1

## Patentansprüche

1. Weichkapsel auf Stärkebasis, umfassend
a) > 40 Gew.-% der trockenen Weichkapsel, nach Abzug des Weichmachers, Stärke,
b) 15 - 70 Gew.-% der trockenen Weichkapsel Weichmacher, und
c) 0,1 - 50 Gew.-% der gesamten Weichkapsel Wasser,
wobei die Weichkapsel miteinander verbundene Partikel destrukturierter Stärke aufweist.

2. Weichkapsel auf Stärkebasis nach Anspruch 1, wobei die miteinander verbundenen Partikel destrukturierter Stärke mindestens bis zur Stufe 2.1 destrukturiert sind, wobei Stärke mit einer Destrukturierung der Stufe 2.1 dadurch charakterisiert ist, dass 40-50 % der Stärkekörner im Polarisationsmikroskop nicht mehr doppelbrechend sind.

3. Weichkapsel auf Stärkebasis nach Anspruch 1 oder 2, wobei die miteinander verbundenen Partikel destrukturierter Stärke höchstens bis zur Stufe 3.6 destrukturiert sind, wobei Stärke mit einer Destrukturierung der Stufe 3.6 dadurch charakterisiert ist, dass höchstens 5 % der Stärkekörner im Polarisationsmikroskop doppelbrechend sind und 70-100 % der Stärkekörner aufgeplatzt sind.

4. Weichkapsel auf Stärkebasis nach einem der vorangehenden Ansprüche 1 bis 3, wobei die Stärke eine Wurzelstärke oder eine Mischung von Wurzelstärken, insbesondere Kartoffelstärke und/oder Tapiocastärke ist.

5. Weichkapsel auf Stärkebasis nach einem der vorangehenden Ansprüche 1 bis 4, wobei die Stärke im nativen Zustand eingesetzt wird.

6. Weichkapsel auf Stärkebasis nach einem der vorangehenden Ansprüche 1 bis 5, wobei die Weichkapsel, bezogen auf die trockene Weichkapsel und nach Abzug des Weichmachers, maximal 50 Gew.-% Verdickungsmittel umfasst.

7. Weichkapsel nach einem der vorangehenden Ansprüche 1 bis 6, wobei die Weichkapsel übliche Zusatz- und Hilfsstoffe umfasst.

8. Weichkapsel auf Stärkebasis nach einem der vorangehenden Ansprüche 1 bis 7, wobei die Weichkapsel eine Matrix aus miteinander verbundenen Partikeln destrukturierter Stärke aufweist.

9. Weichkapsel auf Stärkebasis nach einem der vorangehenden Ansprüche 1 bis 8, wobei die Stärke in der Weichkapselhülle einen Anteil von mindestens 30% aufweist, der nach Lösen der Weichkapsel bei 70°C während 30 min in Form von Partikeln vorliegt und durch Sedimentation zurück gewonnen werden kann.

10. Weichkapsel auf Stärkebasis nach einem der vorangehenden Ansprüche 1 bis 9, wobei die trockene Weichkapselhülle einen Anteil von mindestens 25 Gew.-% Feststoffgehalt aufweist, der nach Lösen der Weichkapsel bei 70°C während 30 min durch Sedimentation zurück gewonnen werden kann.

11. Weichkapsel auf Stärkebasis nach einem der vorangehenden Ansprüche 1 bis 10, wobei das Gewichtsmittel der Molekulargewichtsverteilung der Stärke in der hergestellten Weichkapsel mindestens 500.000 g/mol, vorzugsweise mindestens 2.500.000 g/mol beträgt.

12. Weichkapsel auf Stärkebasis nach einem der vorangehenden Ansprüche 1 bis 11, wobei die Weichkapsel, bezogen auf die trockene Weichkapsel und nach Abzug des Weichmachers, 0 Gew.-% oder maximal 5 Gew.-%, vorzugsweise maximal 3 Gew.-%, Gelatine umfasst.

13. Weichkapsel auf Stärkebasis nach einem der vorangehenden Ansprüche 1 bis 12, wobei Wassergehalt der Weichkapsel maximal 40 Gew.-%, vorzugsweise höchstens 30 Gew.-% beträgt.

14. Weichkapsel auf Stärkebasis nach einem der vorangehenden Ansprüche 1 bis 13, wobei Wassergehalt der Weichkapsel mindestens 1 Gew.-%, vorzugsweise mindestens 5 Gew.-% beträgt.

15. Film zur Herstellung von Weichkapseln auf Stärkebasis nach einem der vorangehenden Ansprüche 1 bis 14.

16. Film auf Stärkebasis, umfassend
a) > 40 Gew.-% des trockenen Films, nach Abzug des Weichmachers, Stärke,
b) 15 - 70 Gew.-% des trockenen Films Weichmacher, und
c) 0,1 - 50 Gew.-% des gesamten Films Wasser,
wobei der Film miteinander verbundene Partikel destrukturierter Stärke aufweist.

## Claims

1. Soft capsule based on starch, comprising
a) > 40 weight percent of the dry soft capsule, after subtracting the plasticizer, starch,
b) 15 - 70 weight percent of the dry soft capsule plasticizer, and
c) 0.1 - 50 weight percent of the total soft capsule water, wherein the soft capsule comprises particles of destructured starch bonded to one another.

2. Soft capsule based on starch according to claim 1, wherein the particles of destructured starch bonded to one another are destructured at least up to stage 2.1, wherein the starch with a destructuring of stage 2.1 is **characterised in that** 40-50 % of the starch grains are no longer birefringent in a polarization microscope.

3. Soft capsule based on starch according to claim 1 or 2, wherein the particles of destructured starch bonded to one another are destructured at most up to stage 3.6, wherein the starch with a destructuring of stage 3.6 is **characterised in that** at most 5 % of the starch grains are birefringent in a polarization microscope and 70-100 % of the starch grains are burst.

4. Soft capsule based on starch according to anyone of the preceding claims 1 to 3, wherein the starch is a root starch or a mixture of root starches, particularly potato starch and/or tapioca starch.

5. Soft capsule based on starch according to anyone of the preceding claims 1 to 4, wherein the starch is used in a native state.

6. Soft capsule based on starch according to anyone of the preceding claims 1 to 5, wherein the soft capsule comprises thickener in an amount of at most 50 weight percent, based on the dry soft capsule and after subtracting the plasticizer.

7. Soft capsule based on starch according to anyone of the preceding claims 1 to 6, wherein the soft capsule comprises conventional additives and adjuvants.

8. Soft capsule based on starch according to anyone of the preceding claims 1 to 7, wherein the soft capsule comprises a matrix of particles of destructured starch bonded to one another.

9. Soft capsule based on starch according to anyone of the preceding claims 1 to 8, wherein the starch in the soft capsule shell, which is present in the form of particles after dissolving the soft capsule for 30 min at 70°C and can be recovered by sedimentation, has a proportion of at least 30%.

10. Soft capsule based on starch according to anyone of the preceding claims 1 to 9, wherein the dry soft capsule shell has a proportion of at least 25 weight percent solids content, which can be recovered after dissolving the soft capsule for 30 min at 70°C by sedimentation.

11. Soft capsule based on starch according to anyone of the preceding claims 1 to 10, wherein the weight-average of the molecular weight distribution of the starch in the produced soft capsule is at least 500,000 g/mol, preferably 2,500,000 g/mol.

12. Soft capsule based on starch according to anyone of the preceding claims 1 to 11, wherein the soft capsule comprises 0 weight percent or at most 5 weight percent, preferably 3 weight percent of gelatin, based on the dry soft capsule and after subtracting the plasticizer.

13. Soft capsule based on starch according to anyone of the preceding claims 1 to 12, wherein water content of the soft capsule is at most 40 weight percent, preferably at least 30 weight percent.

14. Soft capsule based on starch according to anyone of the preceding claims 1 to 13, wherein water content of the soft capsule is at least 1 weight percent, preferably at least 5 weight percent.

15. Film for producing soft capsules based on starch according to anyone of the preceding claims to 14.

16. Film based on starch, comprising
a) > 40 weight percent of the dry film, after subtracting the plasticizer, starch,
b) 15-70 weight percent of the dry film plasticizer, and
c) 0.1-50 weight percent of the total soft capsule water, wherein the film comprises particles of destructured starch bonded to one another.

## Revendications

1. Gélule molle sur base d'amidon, comprenant
a) > 40 % en poids de la gélule molle sèche après retrait du plastifiant d'amidon,
b) de 15 à 70 % en poids de la gélule molle sèche de plastifiant,
c) de 0,1 à 50 % en poids de la gélule molle d'eau
la gélule molle comportant des particules liées les unes aux autres d'amidon déstructuré.

2. Gélule molle sur base d'amidon selon la revendication 1, les particules liées les unes aux autres d'amidon déstructuré étant déstructurées au moins jusqu'au niveau 2.1., l'amidon d'une déstructuration de niveau 2.1 étant **caractérisé en ce que** de 40 à 50 % des granules d'amidon ne sont plus biréfringents au microscope polarisant.

3. Gélule molle sur base d'amidon selon la revendication 1 ou 2, les particules liées les unes aux autres d'amidon déstructuré étant déstructurées au maximum jusqu'au niveau 3.6, l'amidon d'une déstructuration de niveau 3.6 étant **caractérisé en ce qu'**au maximum 5 % des granules d'amidon sont biréfringents au microscope polarisant et de 70 à 100 % des granules d'amidon ont éclaté.

4. Gélule molle sur base d'amidon selon l'une quelconque des revendications 1 à 3 précédentes, l'amidon étant un amidon de tubercules ou un mélange d'amidons de tubercules, notamment de la fécule de pommes de terre et/ou de la fécule tapioca.

5. Gélule molle sur base d'amidon selon l'une quelconque des revendications 1 à 4 précédentes, l'amidon étant mis en œuvre dans l'état natif.

6. Gélule molle sur base d'amidon selon l'une quelconque des revendications 1 à 5 précédentes, la gélule molle comprenant un maximum de 50 % en poids d'agent épaississant, rapportés à la gélule molle et après retrait du plastifiant.

7. Gélule molle sur base d'amidon selon l'une quelconque des revendications 1 à 6 précédentes, la gélule molle comprenant des additifs et excipients usuels.

8. Gélule molle sur base d'amidon selon l'une quelconque des revendications 1 à 7 précédentes, la gélule molle comportant une matrice de particules liées les unes aux autres d'amidon déstructuré.

9. Gélule molle sur base d'amidon selon l'une quelconque des revendications 1 à 8 précédentes, l'amidon comportant dans l'enveloppe de la gélule molle une part d'au moins 30 %, qui après dissolution de la gélule molle à 70 °C pendant 30 mn se présente sous la forme de particules et peut être récupérée par sédimentation.

10. Gélule molle sur base d'amidon selon l'une quelconque des revendications 1 à 9 précédentes, l'enveloppe sèche de la gélule molle comportant une part d'au moins 25 % en poids de teneur en solides, qui après dissolution de la gélule molle à 70 °C pendant 30 mn peut être récupérée par sédimentation.

11. Gélule molle sur base d'amidon selon l'une quelconque des revendications 1 à 10 précédentes, la moyenne pondérale de la distribution des poids moléculaires des gélules molles fabriquées s'élevant à au moins 500.000 g/mole, de préférence à au moins 2.500.000 g/mole.

12. Gélule molle sur base d'amidon selon l'une quelconque des revendications 1 à 11 précédentes, la gélule molle comprenant 0 % en poids ou un maximum de 5 % en poids, de préférence, un maximum 3 % en poids de gélatine, rapportés à la gélule molle sèche et après retrait du plastifiant.

13. Gélule molle sur base d'amidon selon l'une quelconque des revendications 1 à 12 précédentes, la teneur en eau de la gélule molle s'élevant à un maximum de 40 % en poids, de préférence à un maximum de 30 % en poids.

14. Gélule molle sur base d'amidon selon l'une quelconque des revendications 1 à 13 précédentes, la teneur en eau de la gélule molle s'élevant à un minimum de 1 % en poids, de préférence à un minimum de 5 % en poids.

15. Film, destiné à la fabrication de gélules molles sur base d'amidon selon l'une quelconque des revendications 1 à 14 précédentes.

16. Film sur base d'amidon, comprenant
a) > 40 % en poids de la gélule molle sèche après retrait du plastifiant d'amidon,
b) de 15 à 70 % en poids de la gélule molle sèche de plastifiant,
c) de 0,1 à 50 % en poids de la gélule molle d'eau
le film comportant des particules liées les unes aux autres d'amidon déstructuré.
